(19) **Europäisches Patentamt**
**European Patent Office**
**Office européen des brevets**

(11) **EP 3 582 836 B1**

(12) **EUROPEAN PATENT SPECIFICATION**

(45) Date of publication and mention
of the grant of the patent:
**14.06.2023 Bulletin 2023/24**

(21) Application number: **18712486.2**

(22) Date of filing: **16.02.2018**

(51) International Patent Classification (IPC):
***A61M 11/00*** (2006.01)          ***A61M 16/06*** (2006.01)
***A61M 16/20*** (2006.01)

(52) Cooperative Patent Classification (CPC):
**A61M 16/207; A61M 11/00; A61M 16/0677;**
A61M 2202/0208

(86) International application number:
**PCT/US2018/000038**

(87) International publication number:
**WO 2018/151826 (23.08.2018 Gazette 2018/34)**

(54) **SYSTEM FOR GAS DELIVERY INCLUDING GAS CONSERVER**

SYSTEM ZUR GASABGABE MIT GASKONSERVIERER

SYSTÈME DE DISTRIBUTION DE GAZ COMPRENANT UN DISPOSITIF DE CONSERVATION DE GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.02.2017 US 201762459940 P**
**24.02.2017 US 201762463374 P**
**28.02.2017 US 201762464481 P**

(43) Date of publication of application:
**25.12.2019 Bulletin 2019/52**

(73) Proprietor: **L'Air Liquide, Société Anonyme pour l'Etude**
**et l'Exploitation des Procédés Georges Claude**
**75007 Paris (FR)**

(72) Inventors:
• **KRENTLER, Stephen, Bruce**
**Nazareth, PA 18064 (US)**
• **IRR, Hans**
**Nazareth, PA 18064 (US)**
• **TREVENA, Paul, Norman**
**Maidenhead,**
**Berkshire L6 8RA (GB)**

(74) Representative: **Air Liquide**
**L'Air Liquide S.A.**
**Direction de la Propriété Intellectuelle**
**75, Quai d'Orsay**
**75321 Paris Cedex 07 (FR)**

(56) References cited:
WO-A1-01/21240          WO-A1-2006/092635
WO-A1-2017/040978          WO-A2-96/40336
US-B1- 6 484 721

**Description**

**Background**

**Field of the Invention**

The present invention relates to the use of gas conservers in gas delivery systems, more particularly, in gas delivery systems for inhalation of medical oxygen or emergency oxygen as defined by the appended claims.

**Related Art**

Gas conservers are used to provide controlled amounts of gas for inhalation by persons. Two examples of conservers include those for delivering oxygen for patient oxygen therapy and oxygen for airplane crew and/or passengers in low oxygen or low pressure environments aboard aircraft.

Oxygen conservers in the home healthcare market have for many years improved the usable duration of supply vessels (cylinders, tanks, containers, etc.) over continuous flow devices (regulators, flowmeters, liquid oxygen dewars, etc.) while still adequately satisfying the clinical needs of the oxygen patient. These systems typically only provide oxygen to the user during the inhalation portion of a breath, optimally in the first half of the user's inhalation.

Initially most devices were electronic using batteries as a power source. Later, pneumatic device using the pressure in the supply vessel as a power source became the preferred systems due to no need for batteries, smaller size and ease of use. Both types afforded the oxygen patient to ambulate for longer periods of time and/or require less frequent oxygen supply refills.

Weight reduction in aerospace is a constant goal to reduce fuel consumption, increase range and improve safety. Current systems for on-board oxygen provide continuous flow oxygen which limits supply duration, requires the maximum size supply vessel storage space will allow regardless if used which in most flights they are not and can limit aircraft range.

Some home healthcare demand systems have been tried in commercial aviation market with limited acceptance and success. Most were not durable enough for the rigors of the commercial aviation market or provided inadequate interface with the aircraft storage system. US648721 discloses an example of a pneumatic conserving oxygen device.

Therefore it is an object of the invention to provide an apparatus for regulating gas flow with a conserver that does not experience the problems exhibited by conventional gas flow regulation methods and apparatuses.

**Summary**

There is disclosed a pneumatic oxygen conserving system for delivering oxygen for inhalation, comprising tubing that is adapted and configured to receive gas from a source of gas, a conserver in downstream flow communication with said tubing, and a wearable gas distribution device comprising a face mask, a nasal mask, or nasal cannula in downstream flow communication with said conserver that is adapted and configured to deliver oxygen to a person for inhalation thereof, the conserver comprising a main body into which is formed: a vent orifice that opens out of the main body; a slave chamber divided into upper and lower regions by a slave diaphragm; a sensing chamber divided into upper and lower regions by a sensing diaphragm; an inhalation sensing passageway that opens into the main body in upstream flow communication with the sensing chamber lower region; slave chamber inlet passage fluidly communicating between the main body inlet and the slave chamber upper region; a slave chamber outlet passage in flow communication between the slave chamber upper region and the mask or nasal cannula; a timing gas inlet passage in upstream fluid communication with the slave chamber lower region; and a timing gas outlet passage fluidly communicating between the slave chamber lower region, the sensing chamber upper region, and the vent orifice, wherein:

an inhalation gas flow path is comprised of said slave chamber inlet passage, said slave chamber upper region, and said slave chamber outlet passage;
a timing gas flow path is comprised of said timing gas inlet passage, said slave chamber lower region, said timing gas outlet passage, and said vent orifice;
the slave diaphragm is biased to a closed position in which it occludes flow communication between the slave chamber inlet and outlet passages and a flow of oxygen through the inhalation gas flow path is prevented;
the slave diaphragm is moved from its closed position to its open position when a difference in pressure, $P_{slave\ upper}$ - $P_{slave\ lower}$, between the slave chamber upper and lower regions, respectively, exceeds a predetermined differential pressure $P_{slave\ break}$;
the sensing diaphragm is biased in a closed position in which it occludes flow communication between timing gas inlet and outlet passages and prevents a flow of oxygen through the timing gas flow path;

the sensing diaphragm is moved from its closed position to its open position when a difference in pressure, $P_{sensing\ upper} - P_{sensing\ lower}$, between the secondary slave chamber upper and lower regions, respectively, exceeds a predetermined differential pressure $P_{sensing\ break}$; and
the conserver is adapted and configured such that:

when the sensing and slave diaphragms are in their closed positions, inhalation of a person wearing the mask or nasal cannula will cause $P_{sensing\ upper} - P_{sensing\ lower}$ to exceed $P_{sensing\ break}$ and move the sensing diaphragm to its open position, oxygen flows out of the vent orifice via the main body inlet and the timing gas inlet and outlet passages, and an oxygen flow out of the inhalation gas outlet passage is delayed by a time period $\Delta T_1$;
at the expiration of $\Delta T_1$, a decrease in pressure in the timing gas flow path causes $P_{slave\ upper} - P_{slave\ lower}$ to exceed $P_{slave\ break}$ and the slave diaphragm is moved to its open position and oxygen flows through the inhalation gas flow path while the sensing diaphragms remains in its open position for a time period $\Delta T_2$;
at the expiration of $\Delta T_2$, $P_{sensing\ upper} - P_{sensing\ lower}$ drops below $P_{sensing\ break}$ and the sensing diaphragm is moved to its closed position and oxygen is prevented from flowing through the timing gas flow path while oxygen continues to flow through the inhalation gas flow path for a time period $\Delta T_3$;
at the expiration of $\Delta T_3$, an increase in pressure in the timing gas flow path causes $P_{slave\ upper} - P_{slave\ lower}$ to drop below $P_{slave\ break}$ and the slave diaphragm is moved to its closed position and flows of oxygen are prevented through the timing and inhalation gas flow pathways; and
during $\Delta T_2$ and $\Delta T_3$, a bolus of oxygen delivered by the conserver to the mask or nasal cannula is drawn from a reservoir in the tubing.

There is also disclosed an oxygen delivery system comprising an oxygen source, a pressure regulator, and aforementioned pneumatic oxygen conserving system. The pressure regulator is adapted and configured to regulate a pressure of the oxygen source to a lower pressure for delivery into the tubing. The tubing includes an orifice choking a flow of oxygen therethrough that separates the tubing into upstream and downstream portions. The one or both of the inhalation gas inlet and outlet passages includes an orifice choking a flow of oxygen therethrough. The downstream portion of tubing is sized and the orifice(s) of the inhalation gas inlet and outlet passages are dimensioned so as to achieve a predetermined minute volume flow rate of oxygen to the wearable gas distribution device.

There is also disclosed a method of using the aforementioned oxygen delivery system comprising the following steps. The aforementioned oxygen delivery system is provided. The oxygen distribution device is caused to be worn by a person

The pneumatic oxygen conserving system, the oxygen delivery system, and/or the method of using the oxygen delivery system may include one or more of the following aspects:

- the conserver further comprises a main body inlet in fluid communication between the tubing and the inhalation and timing gas flow paths so as to receive a flow of oxygen via the tubing and the main body inlet.
- the conserver further comprises a main body inlet in fluid communication between the tubing and the inhalation gas flow path and the timing gas inlet passage is in downstream flow communication with the tubing so as to receive a flow of oxygen via the tubing but not from the main body inlet.
- the wearable gas distribution device is a face mask or nasal mask comprising an inspiratory valve and an expiratory valve, the conserver is integrated into the face mask or nasal mask, the inhalation gas inlet passage is in fluid communication with a downstream end of the tubing, and the inhalation gas outlet passage opens out into an interior of the face mask or nasal mask.
- the wearable gas distribution device is a nasal cannula and the conserver is disposed in-line with the tubing.
- the conserver is made of a plastic material.
- an altitude adjustment device is disposed downstream of the tubing orifice in-line with the tubing that is adapted and configured to increase or decrease a minute volume flow rate delivered by the conserver when a decrease or increase in ambient pressure caused by an increase or decrease in altitude, respectively.
- the altitude adjustment device comprises a housing having an inlet, an outlet, at least one vent holes on an end thereof that opens out to an exterior of the housing and the ambient atmosphere, and an altitude adjustment diaphragm dividing an interior of the housing into first and second regions, the first region being in flow communication with the housing inlet and outlet, the second region being in flow communication with each of the one or more vent holes, the altitude adjustment diaphragm being biased with a spring into a rest position, a decrease in ambient pressure caused by an increase in altitude causing the diaphragm to move against the biasing of the spring, decrease the volume of the second region, and increase the volume of the first region and consequently increase the volume of the tubing downstream portion.
- the altitude adjustment device comprises a section of the tubing having a flexibility that is greater than remaining sections of the tubing so that a decrease in ambient pressure caused by an increase in altitude causing the section of the tubing comprising the altitude adjustment device to expand outward and increase the volume of the tubing downstream portion

- the oxygen distribution device is a mask and the person is a passenger or crew member of an aircraft.
- the person is a patient receiving oxygen therapy.
- no other chamber is formed in the main body that is in fluid communication with the inhalation gas flow path.
- no electricity is used to initiate a flow of timing gas through the timing gas flow path, initiate a flow of gas through the inhalation gas flow path, move the slave diaphragm to its open closed position, and/or move the sensing diaphragm to its open or closed position.

**Brief Description of the Drawings**

For a further understanding of the nature and objects of the present invention, reference should be made to the following detailed description, taken in conjunction with the accompanying drawings, in which like elements are given the same or analogous reference numbers and wherein:

FIG 1 is a pneumatic schematic visualization of the inventive conserver.
FIG 2 is a perspective view of an embodiment of the inventive oxygen conserving system of the invention including a mask.
FIG 3 is a perspective view of an embodiment of the inventive oxygen conserving system of the invention where the conserver is integrated into a mask.
FIG 4 is a perspective view of an embodiment of the inventive oxygen conserving system of the invention including nasal cannula.
FIG 5 is a perspective view of a mask into which the inventive conserver is integrated.
FIG 6 is a perspective view of an embodiment of the inventive conserver.
FIG 7 is a top plan view of the conserver of **FIG 6.**
FIG 8 is a top-down, perspective, exploded view of the conserver of **FIG 6.**
FIG 9 is a bottom-up, perspective, exploded view of the conserver of **FIG 6.**
FIG 10 is a cross-sectional view of the conserver of **FIG 6** taken along line **I-I.**
FIG 11 is a cross-sectional view of the conserver of **FIG 6** taken along line **II-II**
FIG 12 is a perspective view of another embodiment of the inventive conserver.
FIG 13 a top plan view of the conserver of **FIG 12.**
FIG 14 is a top-down, perspective, exploded view of the conserver of **FIG 12.**
FIG 15 is a bottom-up, perspective, exploded view of the conserver of **FIG 12.**
FIG 16 is a cross-sectional view of the conserver of **FIG 12** taken along line **III-III.**
FIG 17 is a cross-sectional view of the conserver of **FIG 12** taken along line **IV-IV.**
FIG 18 is a perspective view of an embodiment of the inventive oxygen conserving system of the invention including a mask and an altitude adjustment device.
FIG 19 is a perspective view of an embodiment of the inventive oxygen conserving system of the invention including a mask and an altitude adjustment device where the conserver is integrated into the mask.
FIG 20 is a perspective view of a first inventive altitude adjustment device.
FIG 21 is a top plan view of the first altitude adjustment device of **FIG 21.**
FIG 22 is a cross-sectional view of the first altitude adjustment device of **FIG 21** taken along line **V-V.**
FIG 23 is a cross-sectional view of a second altitude adjustment device.

**Description of Preferred Embodiments**

Together with an oxygen source, tubing, and a wearable gas distribution device (a face mask, nasal mask, or nasal cannula), the inventive gas conserver is used to supply a gas to a person only upon their inhalation. The gas is delivered in a bolus very quickly upon detection of the start of inhalation. For use in oxygen therapy, the conserver is adapted and configured for use with cryogenic oxygen systems, oxygen generators, institution wall oxygen gas outlets, portable oxygen systems, and remotely piped oxygen systems. For use aboard aircraft, the conserver is adapted and configured for use with oxygen generators (such as chemical or ceramic oxygen generators or pressure swing adsorption systems) or compressed gas cylinders containing oxygen.

A source of oxygen feeds oxygen at a regulated pressure to the tubing. The term "oxygen" should be construed to include both industrially pure oxygen and also oxygen-rich air.

The tubing includes an orifice in an upstream portion thereof. The portion of the tubing downstream of the orifice and upstream of the conservoir serves as a reservoir for the bolus of oxygen metered out by the conserver. Because this reservoir (i.e., the tubing) is not integrated into the conserver, the conserver may be made with a lower weight and smaller size. In one aspect of the invention, the minute volume flow rate delivered by the conserver may be easily adjusted by adjusting the tubing reservoir volume. Indeed, the inventive device uses the tubing downstream of the orifice as a primary reservoir chamber to accumulate and hold the bolus of oxygen for delivery to the wearer of the mask or nasal cannula until the conserver is triggered by the wearer's inspiratory breath. Along with the conserver's pneumatic timing mechanism, the inside diameter and length of the tube reservoir and the regulated pressure of oxygen supplied by the source determines the maximum volume of the bolus deliverable by the conserver. The pneumatic timing is designed to allow the maximum bolus volume to exit the tube (primary reservoir chamber).

The inventive gas conserver comprises a main body that includes a timing gas flow path and an inhalation gas (oxygen) flow path formed therein. The purpose of the timing gas flow path is to pneumatically open and close the inhalation gas flow path. More particularly, the conserver is adapted and configured such that, after an amount of time $\Delta T_1$ after the flow of oxygen is triggered through the timing gas flow path, a flow of oxygen through an inhalation gas flow path to the mask or nasal cannula is initiated. After passage of an amount of time $\Delta T_2$ after the flow of oxygen through the inhalation gas flow path is triggered, the flow of oxygen through the timing gas flow path is prevented, but the flow through the inhalation gas flow path continues. After passage of an amount of time $\Delta T_3$ after the flow of oxygen through the timing gas flow path is triggered, the flow of oxygen along the inhalation gas flow path is prevented. After passage of an amount of time $\Delta T_4$ after the flow of oxygen through the inhalation gas flow path is prevented, the pressure in the tubing reservoir reaches the regulated pressure provided by the oxygen source. After passage of $\Delta T_3$, the cycle of oxygen delivery may be repeated when the person wearing the mask or nasal cannula draws another breath but, if the breath is drawn before expiration of $\Delta T_4$, the size of the bolus of oxygen delivered will be prorated according to the partial portion of $\Delta T_4$ that has passed.

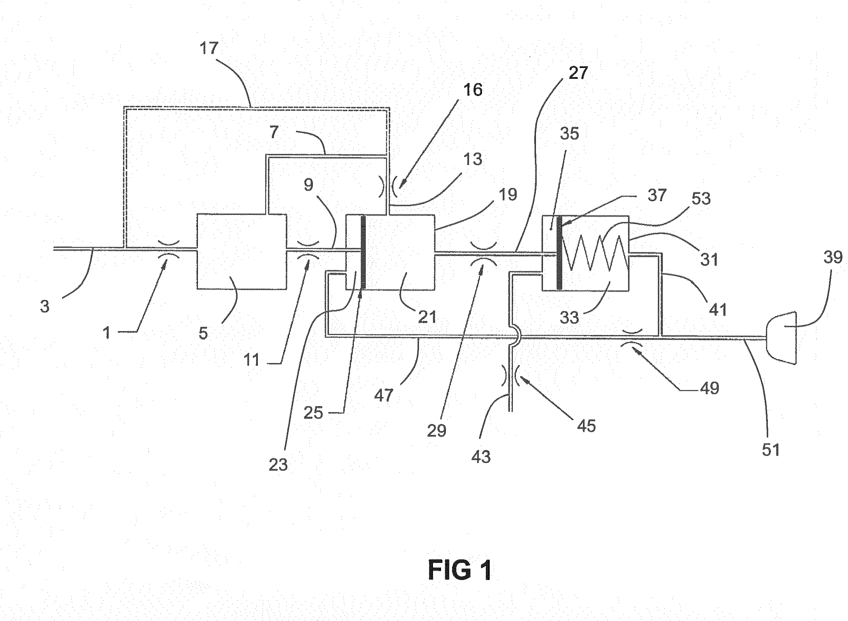

As best illustrated in **FIG 1,** oxygen from a source of oxygen is received into tubing **5** via tubing inlet **3** and tubing orifice **1.** The volume enclosed by the tubing **5** downstream of tubing orifice **1** and upstream of the conserver defines a reservoir of oxygen. Formed within the conserver are a slave chamber **19** and a sensing chamber **31.** A slave diaphragm **25** divides the slave chamber **19** into an upper region **23** and a lower region **21.** Similarly, a sensing diaphragm **37** divides the sensing chamber **31** into an upper region **35** and a lower region **33.**

The tubing **5** supplies oxygen to the lower region **21** of the slave chamber **19** via a slave chamber inlet passage **7** in which is formed a slave chamber inlet orifice **16.** Alternatively, the tubing **5** supplies oxygen to the lower region **21** of the slave chamber **19** via an optional slave chamber inlet passage **17.** In this alternative case, the tubing **5** is provided with two lumens, one of which supplies oxygen to the lower region **21** of the slave chamber **19** and the other which supplies oxygen to the upper region **23** of the slave chamber **19** via the inhalation gas flow path inlet **9.** Otherwise, the tubing **5** has just one lumen supplying oxygen to both the slave chamber inlet passage **7** (including a slave chamber inlet orifice **16)** and inhalation gas flow path inlet passage **9** (including an inhalation gas flow path inlet orifice **11).**

A flow of oxygen, as the timing gas, exits the lower region **21** of the slave chamber **19** via a slave chamber outlet orifice **29** disposed in a slave chamber outlet passage **27.** When in its closed position, the sensing diaphragm **37** (in its closed position) occludes the slave outlet orifice **29** and prevents the timing gas from flowing out of orifice **29.** When a person wearing a mask **39** inhales, the vacuum induced in passages **41, 51** and the lower region **33** of the sensing chamber **31** is sufficient to overcome the biasing of the sensing diaphragm **37** to its closed position by spring **53** and the sensing diaphragm **37** is moved to its open position and no longer occludes orifice **29.** As such, the timing gas flows out of the upper region **35** of the sensing chamber and is vented out of the conserver via a sensing chamber outlet passage **43** having a sensing chamber outlet orifice **45** disposed therein. Consistent with the foregoing, one of ordinary skill in the art will recognize that the timing gas flow path includes slave chamber inlet passage **7** (alternatively optional slave chamber inlet passage **17),** slave chamber inlet orifice **16,** lower region **21** of slave chamber **19,** slave chamber outlet passage **27,** slave chamber outlet orifice **29,** upper region **35** of sensing chamber **31,** sensing chamber outlet passage **43** and sensing chamber outlet orifice **45.**

When in its closed position, the slave diaphragm **25** prevents a flow of oxygen, as the inhalation gas, out of the tube chamber **5** via outlet orifice **11** into the upper region **23** of slave chamber **19.** When in its open position, the inhalation gas flows out of the upper region **23,** into passage **47** and to an interior of the mask **39** via orifice **49.** While **FIG 1** illustrates a single passage **51** fluidly communicating with passages **41, 47** in parallel, passage **51** is optional. In this alternative, both passage **41** and passage **47** separately fluidly communicate with an interior of the mask or nasal cannula **39** (hereinafter "mask"). Consistent with the foregoing, the skilled artisan will recognize that the inhalation gas flow path includes inhalation gas flow path inlet passage **9,** inhalation gas flow path inlet orifice **11,** upper region **23** of slave chamber **19,** passage **47,** orifice **49,** passage **51,** and mask **39.**

In operation, when a person wearing the mask **39** inhales, the pressure difference across the sensing diaphragm **37** increases because a slight vacuum is induced in the lower region **33** of the sensing chamber **31.** This increased pressure difference ($P_{sensing\ upper}$ - $P_{sensing\ lower}$) is sufficient to overcome biasing of the sensing diaphragm **37** into its closed position by spring **53.** Those of ordinary skill in the art will recognize that the biasing ($P_{sensing\ break}$) is a function of the configuration and material of construction of the sensing diaphragm **37** and of the bias applied by the spring **53.** As a result, the timing gas (oxygen) is allowed to flow out of the slave chamber outlet passage **27,** through the upper region **35** of the sensing chamber **31.** The timing gas is thenceforth vented outside the conserver via sensing chamber outlet passage **43** and sensing chamber outlet orifice **45.**

The initiation of the flow of timing gas through the timing gas flow path starts a timer whose expiration (after passage of predetermined time $\Delta T_1$) initiates the flow of inhalation gas (oxygen) through the inhalation gas flow path. After passage of $\Delta T_1$, a decrease in pressure in the lower region **21** of the slave chamber **19** increases the pressure differential ($P_{slave\ upper}$ - $P_{slave\ lower}$) across the slave diaphragm **25** (i.e., pressure in upper region **23** versus pressure in lower region **21**). This pressure differential increases because the flow of timing gas out of the conserver via the sensing chamber outlet passage **43** is relatively uninhibited while the flow of oxygen through the slave chamber inlet orifice **16** is inhibited due to its relatively small hydraulic diameter. As $\Delta T_1$ expires, this increasing pressure differential exceeds the biasing of the slave diaphragm **25** towards its closed position. Those of ordinary skill in the art will understand that, due to the configuration and material of construction of the slave diaphragm **25,** the biasing of the slave diaphragm towards its closed position can be characterized by a predetermined pressure differential ($P_{slave\ break}$). Thus, as $\Delta T_1$ expires, the slave diaphragm **25** is caused to move to its open position.

When the slave diaphragm **25** moves to its open position, the flow of inhalation gas (oxygen) through the inhalation gas flow path is initiated and time $\Delta T_2$ begins. During this time, the decrease in pressure in the upper region **35** of the sensing chamber **31** decreases a pressure differential across the sensing diaphragm **37** ($P_{sensing\ upper}$ - $P_{sensing\ lower}$). As $\Delta T_2$ expires, this decreasing pressure differential decreases to a point where it is longer sufficient to counter the biasing force from spring **53.** As a result, the sensing diaphragm **37** moves back to its closed position and the sensing diaphragm **37** occludes the slave chamber outlet passage **27.**

When the sensing diaphragm **37** moves to its closed position, time $\Delta T_3$ beings, the flow of inhalation gas through the inhalation gas flow path continues, and the flow of timing gas through the timing gas flow path is prevented. During this time, the pressure in the lower region **21** of the slave chamber **19** start to increase. This causes the pressure differential ($P_{slave\ upper}$ - $P_{slave\ lower}$) across the slave diaphragm **25** (i.e., pressure in upper region **23** versus pressure in lower region **21**) to decrease. As $\Delta T_3$ expires, this pressure differential is no longer sufficient to counter the biasing force of the slave diaphragm **25** and the slave diaphragm **25** moves to its closed position. This of course prevents the flow of inhalation gas through the inhalation gas flow path and delivery of the bolus of oxygen is completed

At any time after expiration of $\Delta T_3$, the cycle of initiation of the timing gas flow, the initiation of the inhalation gas flow, the prevention of the timing gas flow, and the prevention of the inhalation gas flow may be repeated when the person wearing the mask **39** draws another breath. If a breath is drawn at the expiration of $\Delta T_4$ or at some point in time after expiration of $\Delta T_4$, the full bolus of oxygen intended to be delivered by the conserver will be delivered to the wearer. On the other hand, if a breath is drawn before passage of $\Delta T_4$, although the pressure in the tubing reservoir may not have reached the regulated pressure provided by the oxygen source, a bolus of oxygen will still be delivered to the wearer. Nevertheless, this bolus will be smaller in comparison to the bolus delivered when a breath is drawn at or after expiration of $\Delta T_4$ because it is prorated against the portion of $\Delta T_4$ that passes before the breath is drawn.

As seen from the foregoing discussion, the conserver allows delivery of bolus of oxygen on demand by the wearer while at the same time preventing the delivery of more than a maximum minute volume flow rate of oxygen to the wearer. The minute volume rate delivered by the conserver may be expressed as:

$$[(\text{bolus vol. delivered during } \Delta T_2 \text{ and } \Delta T_3) + (\text{bolus vol. delivered during } \Delta X)]$$

$$(\Delta T_1 + \Delta T_2 + \Delta T_3 + \Delta X).$$

where $\Delta X$ is the variable amount of time that passes after the flow of oxygen is prevented through the inhalation gas flow path and before a breath is subsequently drawn. Hence, $\Delta X$ depends upon when the wearer draws a breath. Imagine if the wearer draws breath precisely when the reservoir pressure reaches the regulated pressure, $\Delta X = \Delta T_4$ and the intended full bolus of oxygen is delivered. If the wearer instead draws breath after the reservoir pressure reaches the regulated pressure, $\Delta X > \Delta T_4$ and the same intended full bolus of oxygen is again delivered but over a longer period of time. If the wearer instead draws a breath before the reservoir pressure reaches the regulated pressure, $\Delta X < \Delta T_4$ and less than the intended full bolus is delivered because the reservoir pressure has not been "reset". While these boluses are delivered to such a wearer more frequently in comparison to a wearer who waits until at least $\Delta T_4$ passes and the denominator in the above equation is smaller, such boluses are also smaller because they are prorated according to the amount of time that passes before $\Delta T_4$ expires.

Therefore, one can see that the wearer receives a same minute volume rate, regardless of how frequently breaths are drawn, so long as the wearer does not draw a breath before the reservoir pressure is reset. A constant minute volume rate is advantageous because it satisfies not only persons drawing breaths at a moderate rate but also persons taking smaller, more frequent breaths. The conserver also prevents the wastage of oxygen in the event that the wearer draws breaths less frequently than the sum of $\Delta T_1 + \Delta T_2 + \Delta T_3 + \Delta T_4$.

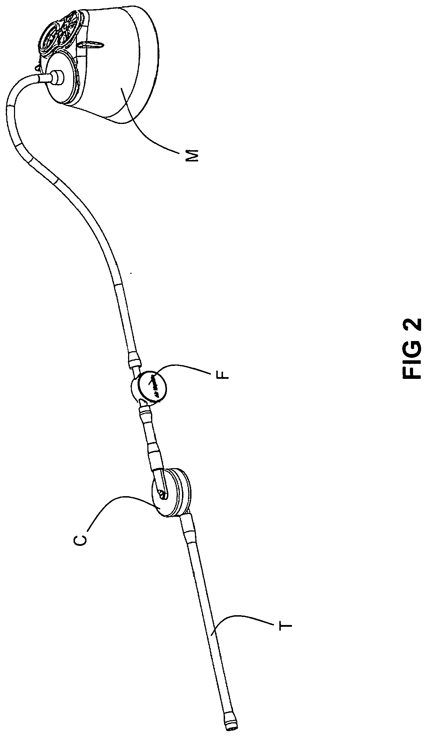

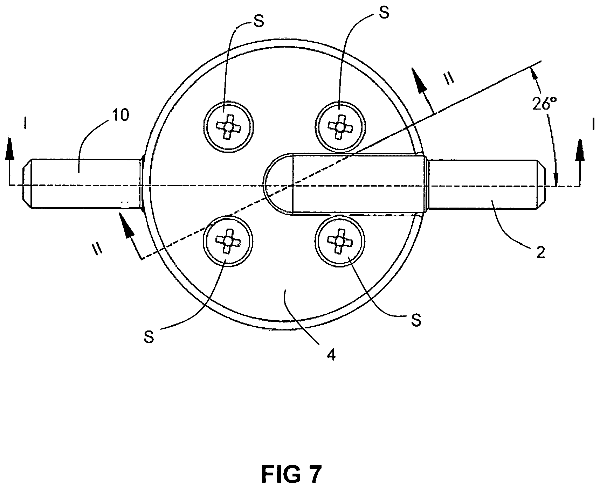

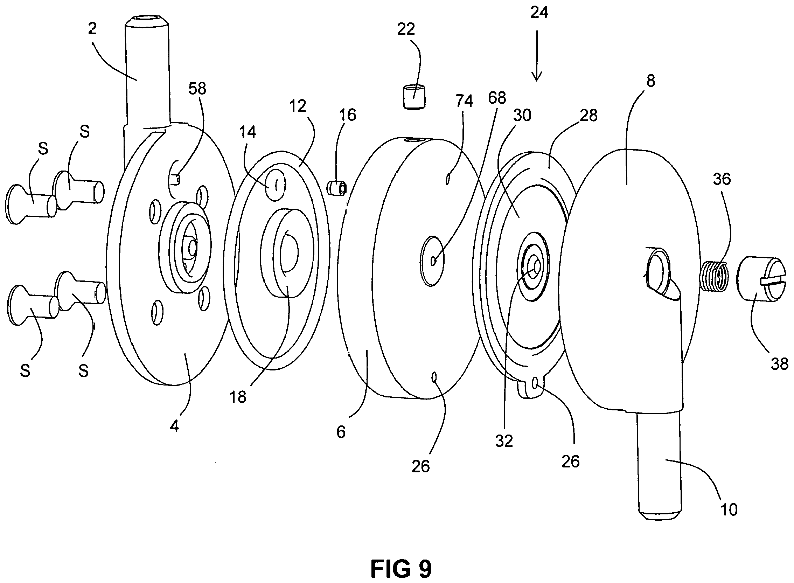

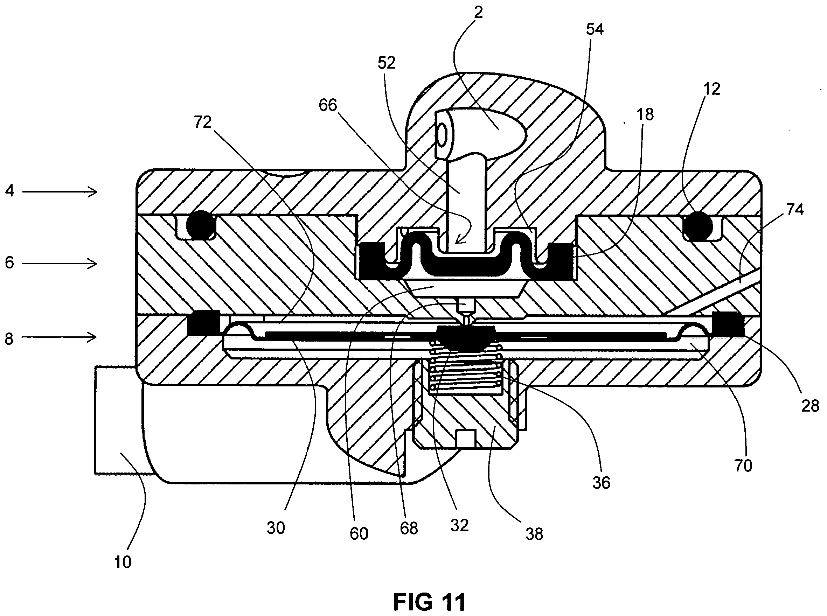

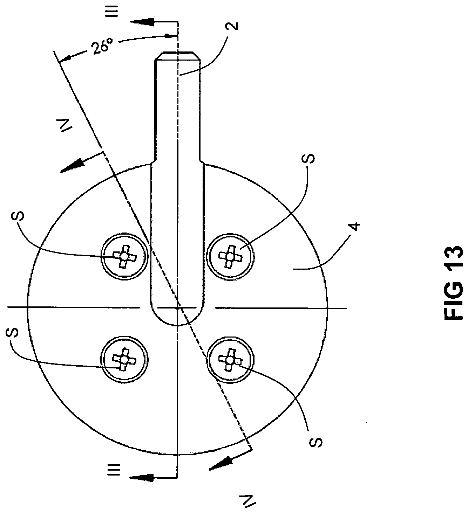

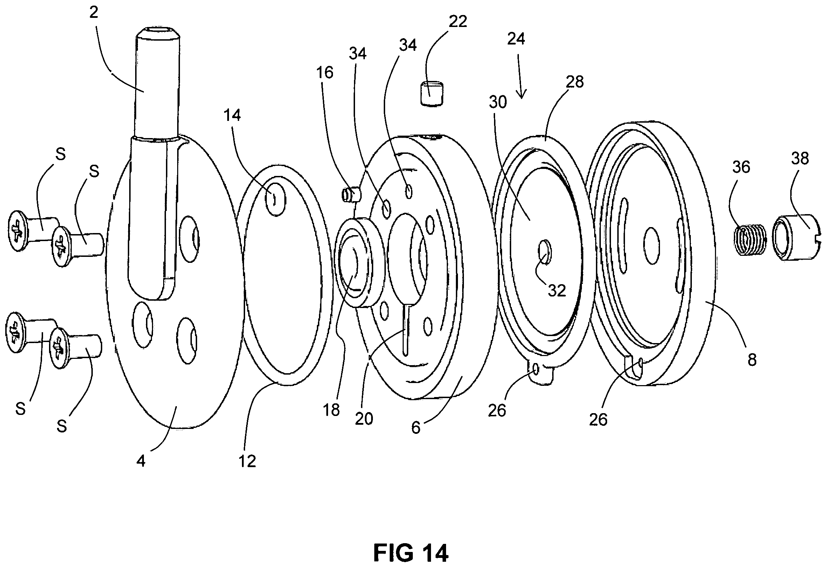

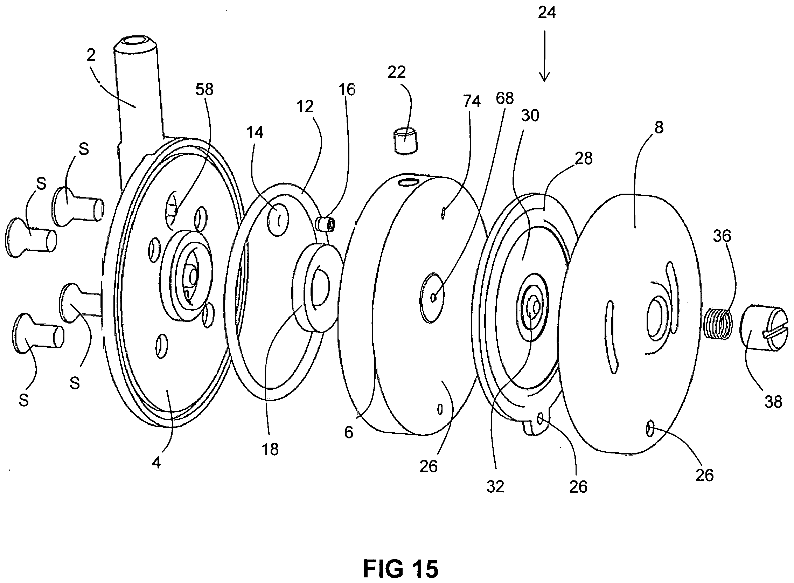

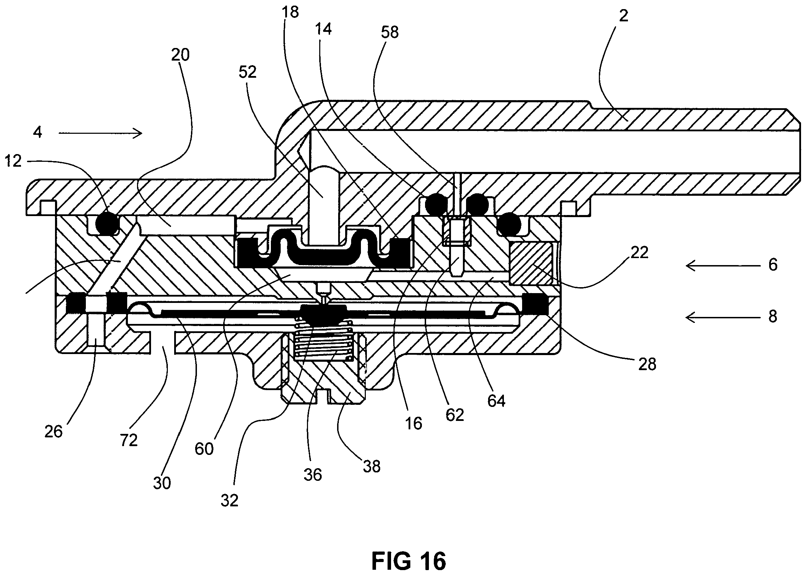

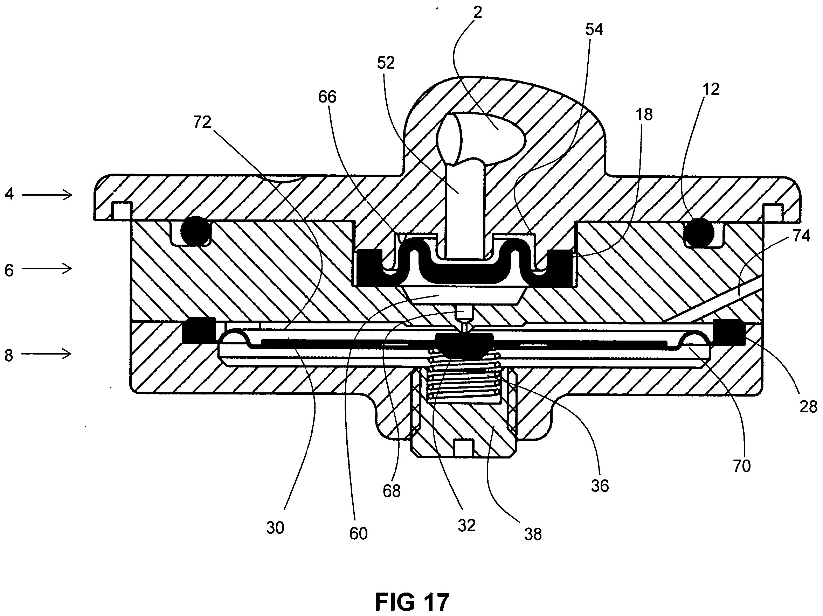

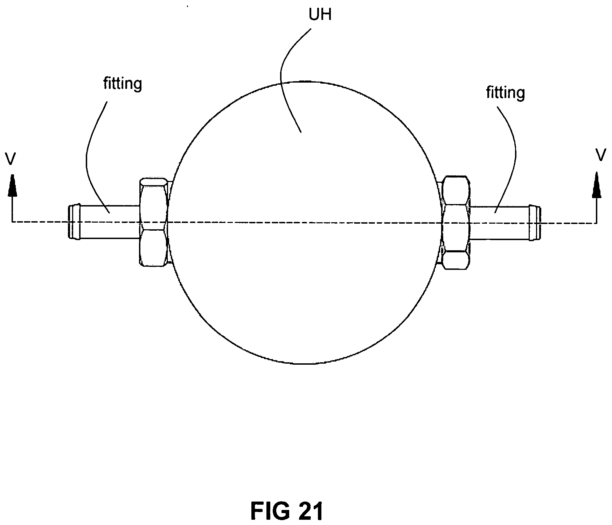

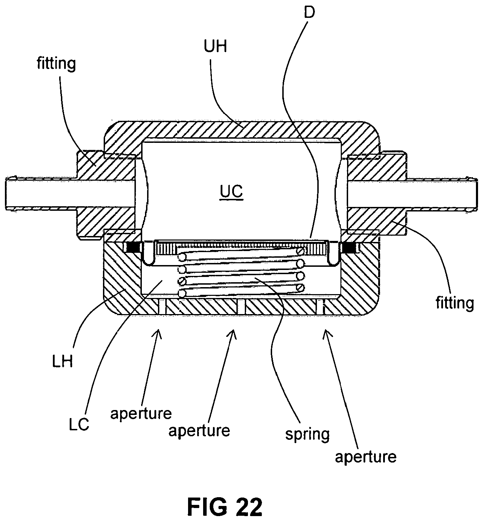

Particular embodiments of the inventive device will now be described with reference to **FIGS 2-22.**

As best shown in **FIG 2,** the oxygen delivery system includes tubing **T,** a conserver **C,** and an optional flow indicator **F.** In this instance, the conserver **C** delivers oxygen to a nasal mask **M.**

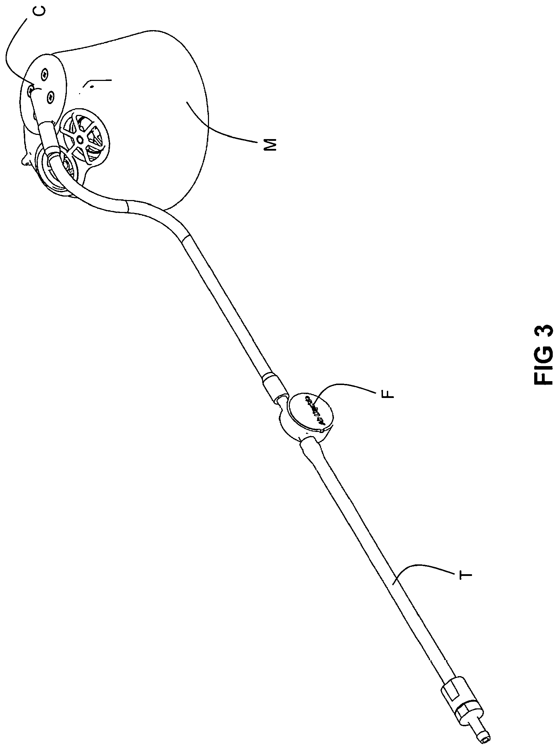

As best illustrated in **FIG 3** and in a different embodiment, the oxygen delivery system includes tubing **T** and a flow indicator **F** used in conjunction with a mask **M.** In contrast to the system of **FIG 2,** however, the conserver **C** is integrated directly into the mask.

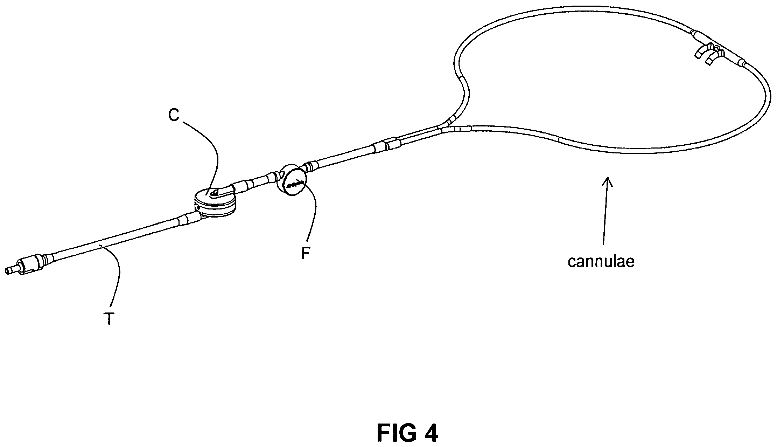

As best shown in **FIG 4** and in yet another embodiment, the oxygen delivery system includes tubing **T,** a conserver **C,** and a flow indicator **F.** In this instance, instead of feeding oxygen to a mask, it is fed to nasal cannula.

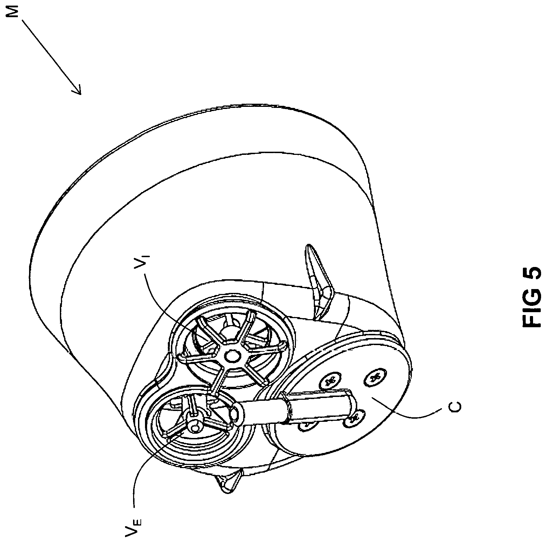

As best illustrated in **FIG 5,** the integrated mask **M** includes an inspiratory valve $V_I$ through which the mask wearer may draw in air and an expiratory valve $V_E$ through which the mask wearer's exhalations may be vented from the mask **M.**

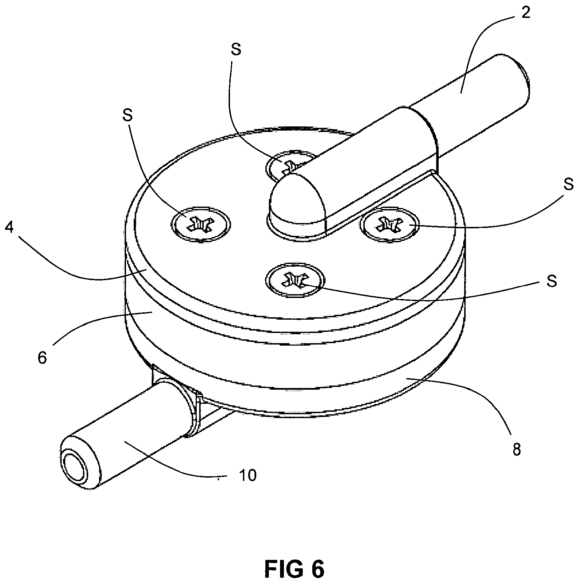

As best shown in **FIGS 6-11** and in the embodiment where the conserver is not integrated into a mask, the conserver includes an upper housing **4** and a lower housing **8** sandwiching a middle housing **6.** At least the conserver housings **4, 6** may be secured to one another with a plurality of screws **S.** One of ordinary skill in the art will recognize, however, that the invention is not limited to such a technique. Ultrasonic welding and/or an adhesive may be used instead. The upper housing **4** includes a main body inlet **2** and the lower housing **8** includes a main body outlet **10.**

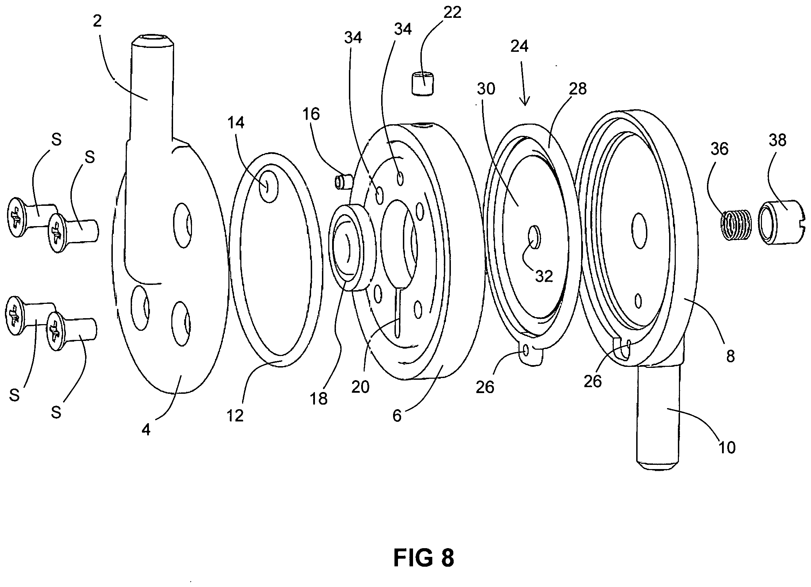

With reference to **FIGS 8-9,** disposed in between the upper and middle housings **4, 6** are o-rings **12,14** and a slave diaphragm **18.** A slave chamber inlet orifice **16** is inserted into any one of a plurality of cavities formed in the middle housing **6** that are adapted and configured to receive the orifice **16** in threaded connection or pressed interference fit. The o-ring **14** or similar seal serves to provide a gas-tight seal at the interface of the upper and middle conserver housings **4, 6** adjacent an upstream end of the orifice **16.** The hollow passage of the orifice **16** is sized to provide a desired length of time during which the timing gas is allowed to flow through the timing gas flow path. A larger orifice **16** will allow a faster flow of the timing gas into the conserver and thus decrease $\Delta T_2$ and $\Delta T_3$. As a result, the volume of the bolus of gas released form the primary chamber tube **5** will be decreased. A smaller orifice **16** will of course have the opposite effect. Optionally, the conserver may be provided with a set of multiple o-rings or seals **14** and orifices **16** where the hollow passage of each orifice **16** has a different hydraulic diameter and each orifice **16** is received by an associated different cavity. In this manner, the conserver may be designed to deliver a variety of minute volume flow rates where the middle housing **6** need only be rotated in order to change the minute volume flow rate. Instead of, or in addition to, a set of multiple o-rings, seals 14, and orifices 16, one out of a variety of minute volume flow rates may be selected by providing a set of tubing inlet orifices having different hydraulic diameters.

Disposed in between the middle and lower housings **6, 8** is the sensing diaphragm **24.** The sensing diaphragm **24** has dual functions. A thickened, peripheral portion **28** provides the function of an o-ring for providing a gas-tight seal between the conserver middle and lower housings **6, 8.** A raised middle portion **32** serves to prevent a flow of timing gas through the timing gas flow path by occluding the slave chamber outlet orifice **68.** An intermediate portion **30** connects the peripheral and middle portions **28, 32.** A spring **36** provides the desired biasing of the sensing diaphragm **24** towards its closed position in which it occludes the slave chamber outlet orifice **68.** The biasing force of the spring **36** may be adjusted by rotating screw cap **38.** Screw cap **38** is threadedly received, in the manner of a nut and bolt, in a correspondingly sized cavity formed in the lower housing **8** to form a gas-tight seal and may be used to adjust the biasing force of the screw **36.**

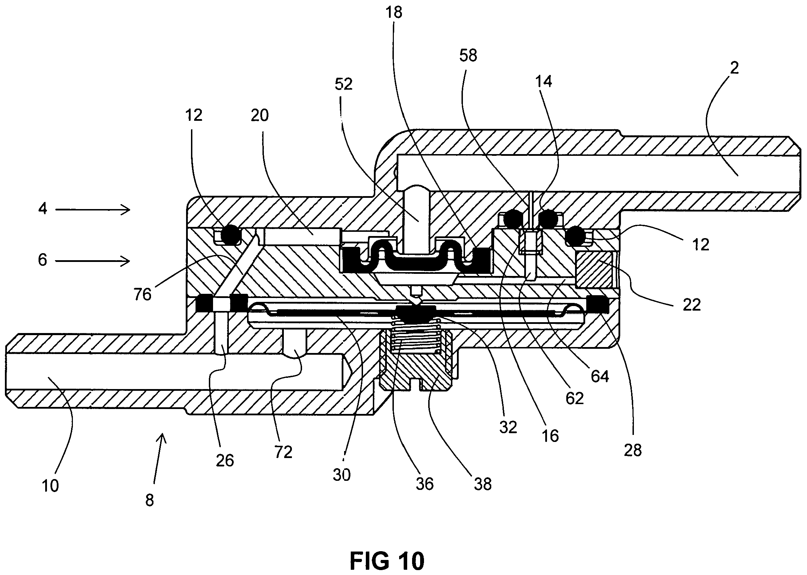

With reference to **FIGS 10-11,** when the wearer of the mask or nasal cannula draws a breath, a slight vacuum induced in a lower region **70** of the sensing chamber (via main body outlet **10** and inhalation sensing passageway **72)** causes the differential pressure across the sensing diaphragm **24** ($P_{sensing\ upper}$ - $P_{sensing\ lower}$) to exceed the bias provided by spring **36** and move the sensing diaphragm **24** to its open position in which the middle portion **32** no longer occludes the slave chamber outlet orifice **68.** As a result of the discontinuation of occlusion, oxygen through the timing gas flow path is no longer prevented and time period $\Delta T_1$ begins.

With respect to the timing gas flow path, oxygen received from the tubing by main body inlet **2** is fed into an upstream end of a slave chamber inlet passage **58.** When the sensing diaphragm is in its open position, the flow continues, in order, through slave chamber inlet orifice **16,** downstream end of slave chamber inlet passage **62,** passage **64,** a lower region **60** of the slave chamber, slave chamber outlet orifice **68,** an upper region **72** of the sensing chamber, and is vented outside the conserver via vent orifice **74.** This flow of the timing gas continues through time periods $\Delta T_1$ and $\Delta T_2$. During time period $\Delta T_1$, the pressure within the timing gas flow path (including the lower region **60** of the slave chamber) decreases. At the expiration of time period $\Delta T_1$, $P_{slave\ upper}$ - $P_{slave\ lower}$ exceeds $P_{slave\ break}$ and the slave diaphragm **18** is moved to its open position and time period $\Delta T_2$ begins.

With respect to the inhalation gas pathway, during time period $\Delta T_1$, an abutment **66** formed on a surface of an upper region **54** of the slave chamber is in contact with the slave diaphragm **18** and prevents a flow of oxygen through the upper region **54.** During time periods $\Delta T_1$ and $\Delta T_2$, oxygen is received from the tubing via inhalation gas inlet passage **52.** The flow continues through an upper region **54** of the slave chamber and enters passages **20, 76, 26.** Together, passages **20, 76, 26** comprise an inhalation gas outlet passage. Because the slave diaphragm **18** has been moved to its open position, the abutment **66** no longer prevents a flow of oxygen through the upper region. During $\Delta T_1$, the pressure in the timing gas flow path decreases, however pressure increases in the upper region **72** of the sensing chamber.

At the expiration of $\Delta T_1$, the pressure in upper region **72** of the sensing chamber has decreased to a point where $P_{sensing\ upper}$ - $P_{sensing\ lower}$ no longer exceeds the biasing force of spring **36** and the sensing diaphragm is moved to its closed position. As such, a flow of oxygen from the slave chamber outlet orifice **68** to the upper region **72** of the sensing chamber is prevented. Consequently, a flow through the timing gas flow path is prevented and the pressure thereof (including in the lower region **60** of the slave chamber) begins to build during time period $\Delta T_3$.

At the expiration of $\Delta T_3$, because of the building pressure in the lower region **60**, $P_{slave\ upper}$ - $P_{slave\ lower}$ goes under $P_{slave\ break}$; the slave diaphragm **18** is moved to its closed position, and contact between abutment **66** and slave diaphragm **18** prevents a flow of oxygen through the upper region **54** of the slave chamber. After passage of $\Delta T_3$, if the wearer of the mask or nasal cannula draws a breath, the cycle repeats with initiation of a flow of oxygen through the timing gas flow path and time period $\Delta T_1$. During $\Delta T_4$, the pressure in the tubing reservoir and the timing and inhalation gas flow paths builds back up to the regulated pressure provided by the oxygen source. Before $\Delta T_4$ expires, any breath drawn by the wearer will result in the delivery of a bolus of oxygen that is prorated according to the degree of completion of $\Delta T_4$.

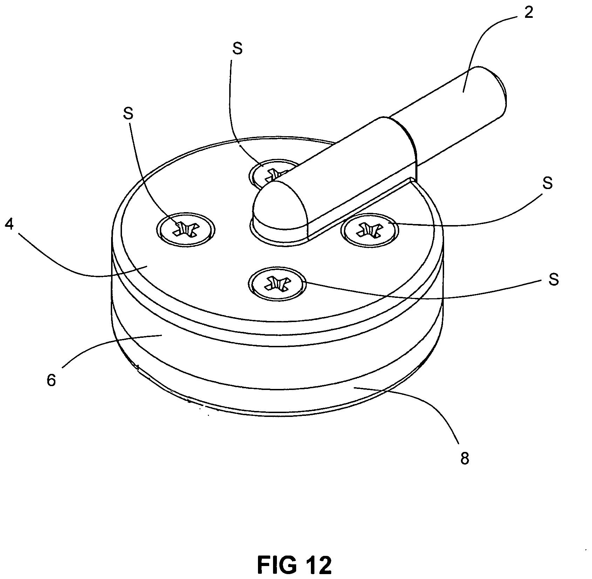

The conserver of the embodiment of **FIGS 12-17** is the same as that of FIGS 6-11 with a few differences. The lower housing 8 is no longer provided with a main body outlet 10. This means that the slave chamber outlet passage made up of passages 20, 76, 26 and the inhalation gas sensing passageway emerge from the main body itself. This particular embodiment is useful when the conserver is integrated into a mask and thus does not need to be connected to any portion of tubing leading to the mask or nasal cannula.

With regard to the description of **FIGS 6-17** above, we note that, instead of the main body having a main body inlet, oxygen may be received by passages **52, 58** directly from the tubing.

In the context of an aeronautical vehicle based system, the invention may also include a device (i.e., an altitude adjustment device). In the case of cabin depressurization, as altitude is increased the ambient air pressure available for breathing in by the wearer via the inspiratory valve of the mask is decreased. Without adjusting for altitude, a conserver whose minute volume flow rate of oxygen is constant will fail to deliver enough oxygen to the wearer to compensate for the decreased amount of oxygen available from the subambient air. If such a constant minute volume flow rate conserer is sized so as to allow a minimum rate at very high altitudes, the conserver will deliver more than the needed rate at relatively lower altitudes and thus waste oxygen and unnecessarily increase the size and thus weight of the source of oxygen.

The altitude adjustment device of the invention acts to increase the minute volume flow rate of oxygen delivered by the conserver during an increase in altitude and decrease the minute volume flow rate of oxygen delivered by the conserver during a decrease in altitude. Any altitude adjustment known in the field of emergency oxygen supplies for aeronautical vehicles may be used in the invention.

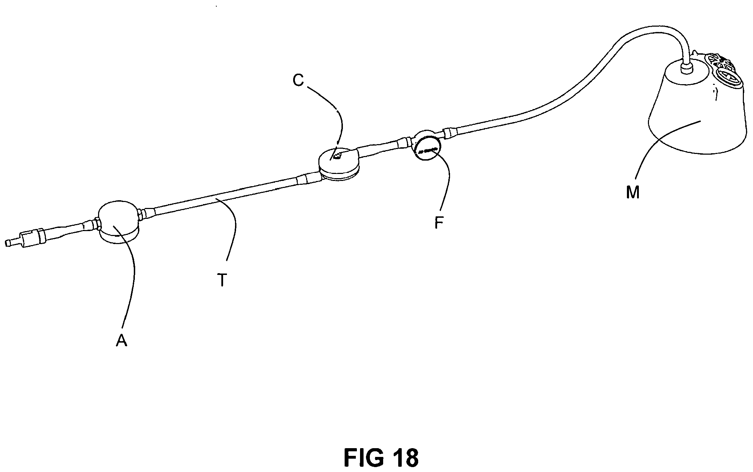

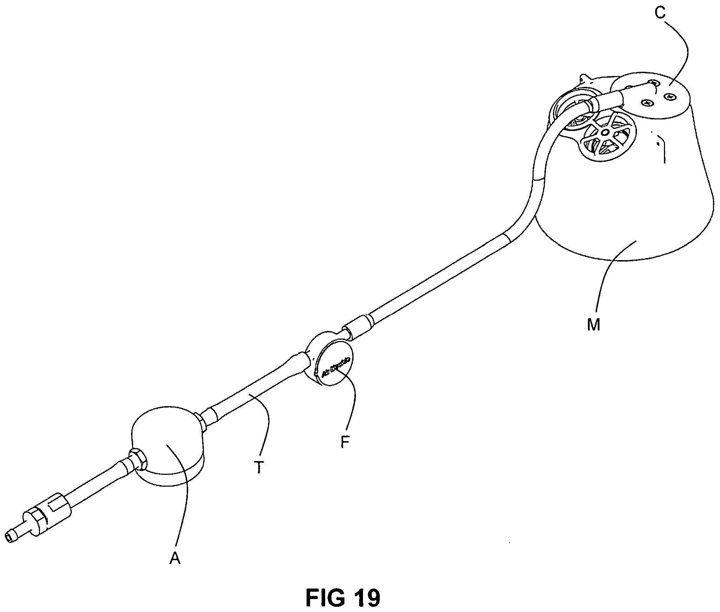

The embodiments of **FIGS 18** and **19** are the same as those of **FIGS 2** and **3,** except that an altitude adjustment device **A** is disposed in-line with the tubing **T** downstream of any inlet orifice of the tubing **T.**

Proposed below are two altitude adjustment devices that adjust the minute volume flow rate of oxygen delivered by the conserver by mechanical means instead of with an electromechanical device.

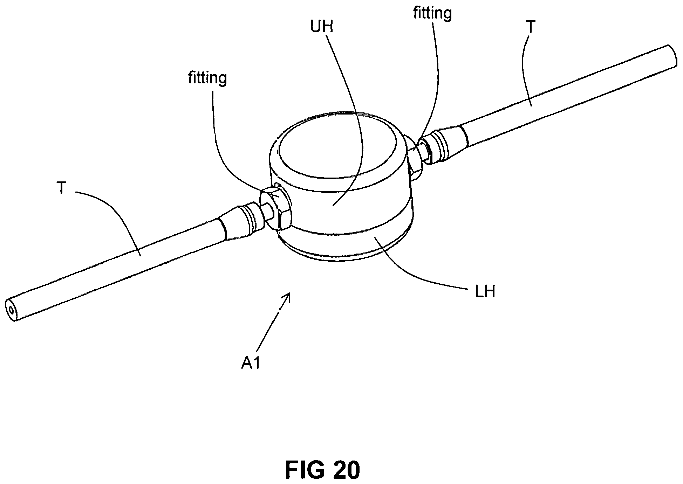

According to the first type of device, and as best shown in **FIGS 20-22,** the altitude adjustment device **A1** acts to increase a volume of the tubing reservoir downstream of the tubing inlet orifice during an altitude increase and decrease a volume of the reservoir during an altitude decrease. The altitude adjustment device **A1** includes an upper housing **UH** secured to a lower housing **LH** in gas-tight fashion. Upstream and downstream ends of the device **A1** are connected in gas-tight fashion to upstream and downstream portions of the tubing **T** via fittings. The interior of the device **A1** is divided into upper and lower cavities **UC, LC** by a diaphragm **D.** The diaphragm is biased towards the upper cavity **UC** by a spring. While the upper cavity **UC** is in fluid communication with the fittings, the lower cavity **LC** is not. The lower cavity **LC** is also not gas-tight in that a plurality of apertures are formed in the lower housing so that the pressure is equalized between the lower cavity **LC** and the ambient atmosphere outside the device. During an increase in altitude, the ambient pressure decreases while the pressure inside the upper cavity **UC** remains the same. As a result, the diaphragm **D** moves downwardly to expand the volume of the upper cavity **UC.** In this manner, the volume of the tubing reservoir is increased so as to increase the minute volume flow rate delivered by the conserver. Conversely, during a decrease in altitude, the ambient pressure increases while the pressure inside the upper cavity **UC** remains the same. As a result, the diaphragm moves upwardly to decrease the volume of the upper cavity **UC.** In this manner, the volume of the tubing reservoir is decreased so as to decrease the minute flow volume rate delivered by the conserver.

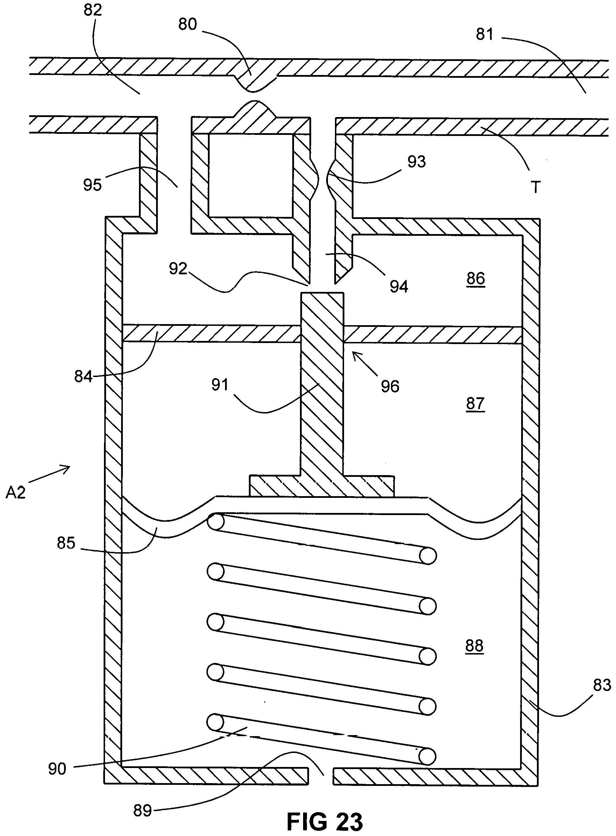

According to the second type of device, and as best shown in **FIG 23,** the altitude adjustment device **A2** acts to increase the amount of oxygen passed through the tubing inlet orifice during an altitude increase and decrease that amount during an altitude decrease. The altitude adjustment device **A2** is secured to the tubing **T** adjacent a tubing inlet orifice **80.** A interior of the altitude adjustment device **A2** enclosed by a housing **83** is divided by a rigid wall 84 and a flexible diaphragm **85** into an upper region **86,** a middle region **87,** and a lower region **88.** The lower region is pressure-equalized to ambient by way of opening **89**. The middle region **87** is gas-tight and filled with a gas at atmospheric pressure. The upper region **86** is also gas-tight (but for fluid communication with tubing T) and pressure-equalized with the pressure in the tubing **T.**

Although **FIG 23** depicts the altitude adjustment device **A2** in its open position, a spring **90** has a biasing force designed to push a rod **91** upward so as to occlude a valve seat **92** when the altitude adjustment device **A2** is at ground level so as to prevent oxygen from an upstream portion **81** of tubing T from flowing through the altitude adjustment device inlet **94** and into upper region **86**.

During an increase in altitude, the pressure difference across the diaphragm **85** increases because, while the middle region **87** is gas-tight and filled with a gas at atmospheric pressure, the lower region **88** is at subambient due to the pressure equalization afforded by opening **89**. The biasing force of the spring **90** is set so that, above a predetermined altitude, the pressure difference across the diaphragm **85** is sufficient to slightly compress the spring **90** and lower the rod **91**. As a result, the valve seat **92** is no longer occluded by the rod **91** and oxygen is allowed to flow from an upstream portion **81** of the tubing **T** to a downstream portion **82** of tubing **T** via inlet **94**, secondary orifice **93**, upper region **86**, and altitude adjustment device outlet **95..** As the gap between the open end of the inlet **94** and the opposing face of the rod **91** increases, the resistance to oxygen flow decreases. The presence of the secondary orifice **93** prevents the oxygen from bypassing the orifice **80**. In this manner, a greater amount of oxygen is allowed to flow into the tubing reservoir downstream of inlet **80**. The minute volume flow rate of oxygen delivered by the conserver is increased, during $\Delta T_2$ and $\Delta T_3$, the pressure in the upper region of the slave chamber will decrease more slowly in comparison to the case where a flow of oxygen is not permitted past orifice **93**. Because the pressure decreases more slowly, a greater amount of oxygen flows through the inhalation gas pathway of the conserver.

As an alternative to the two above-described altitude adjustment devices **A1, A2** shown in **FIGS 20-23,** other mechanical structures adapted and configured to perform the same function may be used. For example, a portion of the tubing may be made of a material having a relatively lower hardness (thus having an inherently greater flexibility) than other portions of the tubing) that expands during an increase in altitude and contracts during a decrease in altitude. As one more example, instead of a spring-biased diaphragm, a stand-alone rolling diaphragm or a piston may be used.

While the inventive system may be used for anything requiring a controlled flow of gas delivered in boluses in a cyclical fashion, the inventive gas demand device is typically used by either a patient in gas therapy, such as oxygen therapy with oxygen, oxygen-enriched gas, or compressed air, or by the crew or passengers of an aircraft during low oxygen and/or pressure conditions.

Whether used by a patient for gas therapy or by aircraft crew in low oxygen and/or low pressure environments, in comparison to conventional gas demand devices, the inventive device has several advantages.

The inventive device reduces the required size and/or weight of an oxygen supply vessel (such as a compressed gas cylinder) and/or increases the time of use in between successive refilling or replacement of the vessel. Decreased size and/or weight are important in the gas therapy context for patients who may experience, muscular weakness, lack of muscle tone, and/or lack of stamina. Decreased size and/or weight will also ordinarily result in decreased costs for the manufacturer, insurer, and/or patient. In contrast to the demand device disclosed in U.S. Patent Application No. 15/255,858, filed September 2, 2016, there is no need to provide a primary chamber (comparable to the tubing reservoir of the instant invention) within the main body. Because the tubing reservoir serves the function of the primary chamber of the device of the '858 application, the volume encompassed by the main body of the instant invention need not be increased to accommodate the volume of such a primary chamber. Because the volume of the main body of the instant invention need not be so large, the material of construction of the main body need not be so heavy.

Decreased size and/or weight are also important in the aerospace context. Aerospace oxygen systems are typically only used in the rare occurrence of a cabin depressurization in the worst case or to provide first aid to an ill passenger. Regardless, every flight must care enough oxygen supply to meet the worst case scenario. Consequently, the weight of these systems on board consumes fuel, reduces payload and range and increases operating costs. Current systems for on-board oxygen provide continuous flow oxygen. Continuous flow limits the duration of time during which the oxygen is supplied. Continuous flow also requires the maximum size supply vessel that the storage space. When an aircraft is used with a same continuous flow system on both short-distance and long-distance flights, the oxygen requirements for the long-distance flight will control. Thus, long-distance flights with a bulkier and/or heavier continuous flow system that decreases fuel consumption. While short-distance flights may utilize a less bulky and/or less heavy continuous flow system, such a system will limit the range of the aircraft on a subsequent flight unless the system is swapped out with a more bulky and/or heavier continuous flow system. By using the inventive gas demand device, the weight can be reduced. Therefore, the aircraft range and/or payload may be increased and fuel consumption decreased. Indeed, in comparison to some conventional systems, the inventive device can reduce the amount of oxygen required on the typical aircraft by as much as 75%. The weight reduction achievable by the inventive device can also improve safety and maintenance costs as well enabling the use of compressed gas cylinders rather than chemical oxygen generators.

The conserver has also been designed to be made of fewer components and lightweight materials, such as plastic and typically high density polyethylene. This simplifies the conserver resulting in better reliability and ease of manufacture. It will also reduce the conserver's weight which will save fuel when utilized in aerospace. This lighter weight can also help maintain a good mask seal on the wearer's face. If a conserver is too heavy (such as conventional conservers), it can pull the mask down and create a leak between the user's face and the mask, thereby causing the inhalation sensing feature of the conserver to fail and not be triggered by the wearer's inspiratory breath. Many conventional devices are electrically powered with a battery and may suffer from power failures, voltage errors and are generally heavier due to the weight of the battery. In contrast, the inventive device functions pneumatically and does not require any electrical power or batteries.

The conserver is designed to be sensitive enough to trigger at a negative pressure less than the cracking pressure of the masks inspiratory valve but less sensitive then similar devices that could cause false triggering if used on a mask. However the design when used with a nasal cannula has good sensitivity due to its close proximity to the user's end of the cannula as opposed to most systems where the conserver is integrated into a regulator on an oxygen cylinder and distant from the user, often 7 feet.

Many conventional devices include features which are freely movable within the device and which may be impacted by the relative position of the device by the force of gravity. For example, some conventional devices may include a ball-type check valve intended to reduce the amount of back pressure created when a pulse of oxygen exits the device and prematurely forces a diaphragm closed. This type of valve is a positional valve that only functions properly when the device is in an orientation where gravity keeps the check valve ball away from its seat. Should the device be inverted the check valve ball will fall to its seat and occlude the passage to the diaphragm, the device may not function since the check valve ball can remain occluded if the inspiration from the user is not great enough to lift the ball from its seat. The amount of negative pressure (< -1.00 cm $H_2O$) typically created by the user at an outlet of such device would most likely not be enough to lift the check valve ball off its seat. In contrast, operation of the inventive device does not depend upon how it is positioned or oriented. In other words, the main diaphragm will not be prematurely closed and operation will not change if the position of the inventive device is changed.

The inventive design is small and easy to use and can be configured and adapted to several modalities such as compressed gas high pressure cylinders, cryogenic oxygen systems, oxygen generators, institution wall oxygen gas outlets, portable oxygen systems and remotely piped oxygen systems.

While some conventional home healthcare gas demand systems have been tried in the commercial aviation market with limited acceptance and success, most were not durable enough for the rigors of the commercial aviation market or provide inadequate interface with the aircraft storage system. The inventive device has been designed with the commercial aviation market in mind in order to overcome the problems experienced by many conventional systems as well as for the healthcare market so as to improve upon current gas demand systems for the healthcare market.

Conventional continuous flow compressed gas systems have a limited use time (for inhalation by the user) that is based upon the volume and pressure of the gas cylinder. Put quite simply, the use time is determined by dividing the mass of gas in the cylinder by the flow rate. In contrast, for the same mass of gas in the gas cylinder, the inventive device extends the use time (for inhalation by the user) because it does not use a continuous flow.

Many conventional gas demand devices tend to be complicated, do not control the volume of gas delivered over time, and do not provide the desired pulse bolus flow curve (i.e., a relatively high peak flow for a short duration) that is best for the person using the device. On the other hand, the inventive device provides the desired bolus flow curve.

Some conventional devices deliver multiple pulses in rapid succession creating a saw tooth gas flow pattern that is depend on constant inhalation and does not control the flow over time. In contrast, the inventive device supplies a bolus of gas upon user demand (i.e., inhalation by the user). Thus, it does not deliver another bolus of gas unless it is demanded by the user.

In comparison to many conventional devices, the inventive device exhibits increased reliability, performance, and ease of use, and a decreased rate of failure caused by uncontrolled user interfaces and real world user conditions.

Most conventional devices depend upon either a back pressure from the gas delivery line or back pressure at an outlet of the device in order to close a main diaphragm and reset its pneumatic circuit. The dependence of a back pressure for closing the main diaphragm is because the last orifice upstream of the outlet is located downstream of a fluidic passage to the diaphragm in question. This particular arrangement will result in a varying back pressure upon the diaphragm; consequently, cause an inconsistent volume per minute delivery.

In contrast, the internal features of the tubing and conserver work together create a timing circuit that is independent of any back pressure exerted onto the sense diaphragm. The sense diaphragm of the inventive conserver resets itself based upon the pneumatic timing circuit and the bias of the spring. Upon movement of the sensing diaphragm to its open position, a "secondary slave chamber" in the upper region of the sensing chamber is created. This secondary slave chamber adds to the timing circuit to ensure the main diaphragm followed by the slave diaphragm does not close before the tubing reservoir volume and pressure is depleted. This is important to ensure any back pressure on the sense diaphragm does not affect the timing and the minute volume is consistent across typical range of breath rates.

An ongoing challenge for most conventional pneumatic demand devices is the ability to be sensitive enough for the person with slow shallow breaths to trigger the device without the device being over sensitive to variations in the gas inlet pressure resulting in the device to self-cycle (auto-pulse). Overly complicated designs exacerbate this sensitivity problem since they magnify the amplitude of any pressure deviations from the specified regulated pressure.

In contrast, the design of the pneumatic circuit of the inventive conserver is simplified, so the amplification of pressure-sensitivity experienced by many conventional devices is significantly dampened in the inventive device. The simplified design of the pneumatic circuit also increases the ease of manufacturing, reduces component count and improves performance. To put a finer point on this assertion, the geometry of the components that make the slave chamber and sensing chamber and the orifices formed in the main body of the conserver are designed to reduce the quantity of components and the cost of the components for manufacturing the device. For example, the device of US 7,089,938 may use as many as 22 components making up the pneumatic circuit while the inventive device may use as few as 15 components.

Legends

| A | first altitude adjustment device |
|---|---|
| C | conserver |
| D | diaphragm for first altitude adjustment device |
| F | flow indicator |
| LC | lower cavity |
| LH | lower housing of altitude adjustment device |
| M | mask |
| S | screw |
| T | tubing |
| UC | upper cavity |
| UH | upper housing of altitude adjustment device |
| $V_E$ | expiratory valve |
| $V_I$ | inspiratory valve |
| 1 | tubing orifice |
| 2 | main body inlet |
| 3 | tubing inlet |
| 4 | upper conserver housing |
| 5 | tubing downstream of tubing orifice |
| 6 | middle conserver housing |
| 7 | slave chamber inlet passage |
| 8 | lower conserver housing |
| 9 | inhalation gas flow path inlet passage |
| 10 | main body outlet |
| 11 | inhalation gas flow path inlet orifice |
| 12 | o-ring |
| 14 | o-ring |
| 16 | slave chamber inlet orifice |
| 17 | optional slave chamber inlet passage |
| 18 | slave diaphragm |

(continued)

| 19 | slave chamber |
|----|---------------|
| 20 | Inhalation gas passage |
| 21 | lower region of slave diaphragm |
| 22 | plug |
| 23 | upper region of slave diaphragm |
| 24 | sense diaphragm |
| 25 | slave diaphragm |
| 26 | inhalation gas passage |
| 27 | slave chamber outlet passage |
| 28 | peripheral portion of sensing diaphragm |
| 29 | slave chamber outlet orifice |
| 30 | middle portion of sense diaphragm |
| 31 | sensing chamber |
| 32 | raised portion of sense diaphragm |
| 33 | lower region of sensing chamber |
| 35 | upper region of sense chamber |
| 36 | spring |
| 37 | sense diaphragm |
| 38 | screw cap |
| 39 | mask |
| 41 | inhalation sense Passage |
| 43 | sensing chamber outlet passage |
| 45 | upper region of sensing chamber outlet orifice |
| 47 | inhalation gas passage |
| 51 | inhalation gas and sense passage |
| 52 | inhalation gas inlet passage |
| 53 | spring |
| 58 | upstream end of slave chamber inlet orifice |
| 60 | lower region of the slave chamber |
| 62 | downstream end of slave chamber inlet orifice |
| 64 | passage |
| 66 | inhalation gas flow path inlet orifice |
| 68 | slave chamber outlet orifice |
| 70 | lower region of sensing chamber |
| 72 | inhalation sense passageway |
| 74 | upper region of sense chamber outlet orifice |
| 76 | passage |

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as fall within the broad scope of the appended claims. The present invention may suitably comprise, consist or consist essentially of the elements disclosed and may be practiced in the absence of an element not disclosed. Furthermore, if there is language referring to order, such as first and second, it should be understood in an exemplary sense and not in a limiting sense. For example, it can be recognized by those skilled in the art that certain steps can be combined into a single step.

The singular forms "a", "an" and "the" include plural referents, unless the context clearly dictates otherwise.

"Comprising" in a claim is an open transitional term which means the subsequently identified claim elements are a nonexclusive listing i.e. anything else may be additionally included and remain within the scope of "comprising." "Comprising" is defined herein as necessarily encompassing the more limited transitional terms "consisting essentially of" and "consisting of"; "comprising" may therefore be replaced by "consisting essentially of" or "consisting of" and remain within the expressly defined scope of "comprising".

"Providing" in a claim is defined to mean furnishing, supplying, making available, or preparing something. The step may be performed by any actor in the absence of express language in the claim to the contrary.

Optional or optionally means that the subsequently described event or circumstances may or may not occur. The description includes instances where the event or circumstance occurs and instances where it does not occur.

Ranges may be expressed herein as from about one particular value, and/or to about another particular value. When such a range is expressed, it is to be understood that another embodiment is from the one particular value and/or to the other particular value, along with all combinations within the range.

**Claims**

**1.** An oxygen delivery system comprising an oxygen source, a pressure regulator, and a pneumatic oxygen conserving system for delivering oxygen for inhalation, comprising tubing (5) that is adapted and configured to receive gas from a source of gas, a conserver (C) in downstream flow communication with said tubing (5), and a wearable gas distribution device comprising a face mask (M), a nasal mask, or nasal cannula in downstream flow communication with said conserver that is adapted and configured to deliver oxygen to a person for inhalation thereof, the conserver comprising a main body into which is formed: a vent orifice (45) that opens out of the main body; a slave chamber (19) divided into upper (23) and lower (21) regions by a slave diaphragm (25); a sensing chamber (31) divided into upper (35) and lower (33) regions by a sensing diaphragm (37); an inhalation sensing passageway that opens into the main body in upstream flow communication with the sensing chamber lower region (33); a slave chamber inlet passage (9) fluidly communicating between the main body inlet (3) and the slave chamber upper region (23); a slave chamber outlet passage (47) in flow communication between the slave chamber upper region (23) and the mask or nasal cannula; a timing gas inlet passage in upstream fluid communication with the slave chamber lower region (21); and a timing gas outlet passage fluidly communicating between the slave chamber lower region (21), the sensing chamber upper region (35), and the vent orifice (45), wherein:

an inhalation gas flow path is comprised of said slave chamber inlet passage (9), said slave chamber upper region (23), and said slave chamber outlet passage (47);
a timing gas flow path is comprised of said timing gas inlet passage, said slave chamber lower region (21), said timing gas outlet passage, and said vent orifice (45);
the slave diaphragm (25) is biased to a closed position in which it occludes flow communication between the slave chamber inlet (9) and outlet (47) passages and a flow of oxygen through the inhalation gas flow path is prevented;
the slave diaphragm (25) is moved from its closed position to its open position when a difference in pressure, $P_{slave\ upper} - P_{slave\ lower}$, between the slave chamber upper (23) and lower (21) regions, respectively, exceeds a predetermined differential pressure $P_{slave\ break}$;
the sensing diaphragm (37) is biased in a closed position in which it occludes flow communication between timing gas inlet and outlet passages (?) and prevents a flow of oxygen through the timing gas flow path;
the sensing diaphragm (37) is moved from its closed position to its open position when a difference in pressure, $P_{sensing\ upper} - P_{sensing\ lower}$, between the secondary slave chamber upper and lower regions, respectively, exceeds a predetermined differential pressure $P_{sensing\ break}$; and
the conserver is adapted and configured such that:

when the sensing (37) and slave (25) diaphragms are in their closed positions, inhalation of a person wearing the mask or nasal cannula will cause $P_{sensing\ upper} - P_{sensing\ lower}$ to exceed $P_{sensing\ break}$ and move the sensing diaphragm to its open position, oxygen flows out of the vent orifice (45) via the main body inlet (3) and the timing gas inlet and outlet passages, and an oxygen flow out of the inhalation gas outlet passage is delayed by a time period $\Delta T_1$;

at the expiration of $\Delta T_1$, a decrease in pressure in the timing gas flow path causes $P_{slave\ upper}$ - $P_{slave\ lower}$ to exceed $P_{slave\ break}$ and the slave diaphragm (25) is moved to its open position and oxygen flows through the inhalation gas flow path while the sensing diaphragms (37) remains in its open position for a time period $\Delta T2$;

at the expiration of $\Delta T_2$, $P_{sensing\ upper}$ - $P_{sensing\ lower}$ drops below $P_{sensing\ break}$ and the sensing diaphragm (37) is moved to its closed position and oxygen is prevented from flowing through the timing gas flow path while oxygen continues to flow through the inhalation gas flow path for a time period $\Delta T_3$;

at the expiration of $\Delta T_3$, an increase in pressure in the timing gas flow path causes $P_{slave\ upper}$ - $P_{slave\ lower}$ to drop below $P_{slave\ break}$ and the slave diaphragm (25) is moved to its closed position and flows of oxygen are prevented through the timing and inhalation gas flow pathways; and

during $\Delta T_2$ and $\Delta T_3$, a bolus of oxygen delivered by the conserver to the mask or nasal cannula is drawn from a reservoir in the tubing wherein the pressure regulator is adapted and configured to regulate a pressure of the oxygen source to a lower pressure for delivery into the tubing (5), the tubing (5) includes an orifice choking a flow of oxygen therethrough that separates the tubing into upstream and downstream portions, the one or both of the inhalation gas inlet and outlet passages includes an orifice choking a flow of oxygen therethrough, and the downstream portion of tubing is sized and the orifice(s) of the inhalation gas inlet and outlet passages are dimensioned so as to achieve a predetermined minute volume flow rate of oxygen to the wearable gas distribution device, the tubing (5) including a tubing orifice (1) to receive oxygen from the oxygen source via a tubing inlet (3), a volume enclosed by the tubing (5), downstream of the tubing orifice (1) and upstream the conserver, defining a tubing reservoir for oxygen.

2. The oxygen delivery system of claim 1, wherein the conserver further comprises a main inlet (2) in flow communication between the tubing (5) and the inhalation and timing gas inlet passages so as to receive flows of oxygen via the tubing (5) and the main body inlet (2).

3. The oxygen delivery system of claim 1, wherein the conserver further comprises a main inlet in flow communication between the tubing (5) and the inhalation and timing gas inlet passages and the timing gas inlet passage is in downstream flow communication with the tubing (5) so as to receive a flow of oxygen via the tubing (5) but not from the main body inlet.

4. The oxygen delivery system of claim 1, wherein the wearable gas distribution device is a face mask or nasal mask comprising an inspiratory valve and an expiratory valve, the conserver is integrated into the face mask or nasal mask, the inhalation and timing gas inlet passages are in fluid communication with a downstream end of the tubing (5), and the inhalation gas outlet passage opens out into an interior of the face mask or nasal mask.

5. The oxygen delivery system of claim 1, wherein the wearable gas distribution device is nasal cannula and the conserver is disposed in-line with the tubing.

6. The oxygen conserving system wherein the conserver is made of a plastic material.

7. The oxygen conserving system, further comprising an altitude adjustment device (A1, A2) disposed downstream of the tubing orifice in-line with the tubing that is adapted and configured to keep the predetermined minute volume flow rate constant when a decrease or increase in ambient pressure caused by an increase or decrease in altitude, respectively.

8. The oxygen delivery system of claim 1, further comprising an altitude adjustment device (A1, A2) disposed downstream of the tubing orifice in-line with the tubing (5) that is adapted and configured to increase or decrease a minute volume flow rate delivered by the conserver when a decrease or increase in ambient pressure, respectively, is caused by an increase or decrease in altitude, respectively.

9. The oxygen delivery system of claim 8, wherein the altitude adjustment device comprises a housing (UH, LH) having an inlet, an outlet, at least one vent holes on an end thereof that opens out to an exterior of the housing and the ambient atmosphere, and an altitude adjustment diaphragm (D) dividing an interior of the housing into first and second regions, the first region being in flow communication with the housing inlet and outlet, the second region being in flow communication with each of the one or more vent holes, the altitude adjustment diaphragm being biased with a spring into a rest position, a decrease in ambient pressure caused by an increase in altitude causing the diaphragm to move against the biasing of the spring, decrease the volume of the second region, and increase the volume of the first region and consequently increase the volume of the tubing downstream portion.

10. The oxygen delivery system of claim 8, wherein the altitude adjustment device comprises a section of the tubing (5) having a flexibility that is greater than remaining sections of the tubing (5) so that a decrease in ambient pressure caused by an increase in altitude causing the section of the tubing comprising the altitude adjustment device to expand outward and increase the volume of the tubing downstream portion

11. The oxygen delivery system of claim 1, wherein the altitude adjustment device is adapted and configured to increase an amount of oxygen flowing into the tubing inlet orifice during an increase in altitude and decrease an amount of oxygen flowing into the tubing inlet orifice during a decrease in altitude.

**Patentansprüche**

1. Sauerstoffabgabesystem, umfassend eine Sauerstoffquelle, einen Druckregler und ein pneumatisches Sauerstoffkonservierungssystem zur Abgabe von Sauerstoff zur Inhalation, umfassend Schlauchmaterial (5), das eingerichtet und ausgestaltet ist, um Gas von einer Gasquelle zu empfangen, einen Konservierer (C) in nachgeschalteter Strömungskommunikation mit dem Schlauchmaterial (5), und eine tragbare Gasverteilungsvorrichtung, umfassend eine Gesichtsmaske (M), eine Nasenmaske oder Nasenkanüle, in nachgeschalteter Strömungskommunikation mit dem Konservierer, die eingerichtet und ausgestaltet ist, um Sauerstoff an eine Person abzugeben, damit diese ihn inhaliert, wobei der Konservierer einen Hauptkörper umfasst, in den ausgebildet ist:

   eine Lüftungsöffnung (45), die sich aus dem Hauptkörper heraus öffnet;
   eine Nebenkammer (19), die durch ein Nebendiaphragma (25) in obere (23) und untere (21) Regionen unterteilt wird;
   eine Abfühlkammer (31), die durch ein Abfühldiaphragma (37) in obere (35) und untere (33) Regionen unterteilt wird;
   einen Inhalationsabfühldurchgang, der sich in vorgeschalteter Strömungskommunikation mit der unteren Region (33) der Abfühlkammer in den Hauptkörper öffnet;
   einen Nebenkammereinlassdurchlass (9), der fließtechnisch zwischen dem Hauptkörpereinlass (3) und der oberen Region (23) der Nebenkammer kommuniziert;
   einen Nebenkammerauslassdurchlass (47) in Strömungskommunikation zwischen der oberen Region (23) der Nebenkammer und der Maske oder Nasenkanüle;
   einen Zeitwahlgaseinlassdurchlass in vorgeschalteter Fließkommunikation mit der unteren Region (21) der Nebenkammer; und
   einen Zeitwahlgasauslassdurchlass, der fließtechnisch zwischen der unteren Region (21) der Nebenkammer, der oberen Region (35) der Abfühlkammer und der Lüftungsöffnung (45) kommuniziert, wobei:

      ein Inhalationsgasströmungspfad aus dem Nebenkammereinlassdurchlass (9), der oberen Region (23) der Nebenkammer und dem Nebenkammerauslassdurchlass (47) zusammengesetzt ist;
      ein Zeitwahlgasströmungspfad aus dem Zeitwahlgaseinlassdurchlass, der unteren Region (21) der Nebenkammer, dem Zeitwahlgasauslassdurchlass und der Lüftungsöffnung (45) zusammengesetzt ist;
      das Nebendiaphragma (25) auf eine geschlossene Position vorgespannt ist, in der es die Strömungskommunikation zwischen dem Nebenkammereinlassdurchlass (9) und Nebenkammerauslassdurchlass (47) versperrt, und eine Sauerstoffströmung durch den Inhalationsgasströmungspfad verhindert wird;
      das Nebendiaphragma (25) aus seiner geschlossenen Position in seine offene Position bewegt wird, wenn eine Druckdifferenz, $P_{\text{Neben oben}} - P_{\text{Neben unten}}$, zwischen der oberen Region (23) und der unteren Region (21) der Nebenkammer einen vorbestimmten Differentialdruck $P_{\text{Neben Durchbruch}}$ übersteigt;
      das Abfühldiaphragma (37) in einer geschlossenen Position vorgespannt ist, in der es die Strömungskommunikation zwischen dem Zeitwahlgaseinlassdurchlass und Zeitwahlgasauslassdurchlass (?) versperrt und eine Sauerstoffströmung durch den Zeitwahlgasströmungspfad verhindert;
      das Abfühldiaphragma (37) aus seiner geschlossenen Position in seine offene Position bewegt wird, wenn eine Druckdifferenz, $P_{\text{Abfühl oben}} - P_{\text{Abfühl unten}}$, zwischen den oberen und unteren Regionen der sekundären Nebenkammer einen vorbestimmten Differentialdruck $P_{\text{Abfühl Durchbruch}}$ übersteigt; und
      der Konservierer so eingerichtet und ausgestaltet ist, dass:

         wenn das Abfühldiaphragma (37) und das Nebendiaphragma (25) in ihren geschlossenen Positionen sind, die Inhalation einer Person, welche die Maske oder Nasenkanüle trägt, bewirkt, dass $P_{\text{Abfühl oben}}$ - $P_{\text{Abfühl unten}}$ $P_{\text{Abfühl Durchbruch}}$ übersteigt und das Abfühldiaphragma in seine offene Position bewegt wird, Sauerstoff aus dem Lüftungsloch (45) über den Hauptkörpereinlass (3) und den Zeitwahlgaseinlassdurchlass und den Zeitwahlgasauslassdurchlass strömt, und eine Sauerstoffströmung aus dem Inhalationsgasauslassdurchlass um einen Zeitraum $\Delta T_1$ verzögert wird;

bei Ablauf von $\Delta T_1$ eine Abnahme des Drucks in dem Zeitwahlgasströmungspfad bewirkt, dass $P_{\text{Neben oben}}$ - $P_{\text{Neben unten}}$ $P_{\text{Neben Durchbruch}}$ überschreitet und das Nebendiaphragma (25) in seine offene Position bewegt wird und Sauerstoff durch den Inhalationsgasströmungspfad strömt, während das Abfühldiaphragma (37) für einen Zeitraum $\Delta T_2$ in seiner offenen Position bleibt;

bei Ablauf von $\Delta T_2$ $P_{\text{Abfühl oben}}$ - $P_{\text{Abfühl unten}}$ auf unter $P_{\text{Abfühl Durchbruch}}$ sinkt und das Abfühldiaphragma (37) in seine geschlossene Position bewegt wird und verhindert wird, dass Sauerstoff durch den Zeitwahlgasströmungspfad strömt, während Sauerstoff für einen Zeitraum $\Delta T_3$ weiter durch den Inhalationsgasströmungspfad strömt;

nach Ablauf von $\Delta T_3$ ein Druckanstieg in dem Zeitwahlgasströmungspfad bewirkt, dass $P_{\text{Neben oben}}$ - $P_{\text{Neben unten}}$ unter $P_{\text{Neben Durchbruch}}$ sinkt und das Nebendiaphragma (25) in seine geschlossene Position bewegt wird und Sauerstoffströme durch die Zeitwahl- und Inhalationsgasströmungspfade hindurch verhindert werden; und

während $\Delta T_2$ und $\Delta T_3$ ein Sauerstoffbolus, der durch den Konservierer an die Maske oder Nasenkanüle abgegeben wird, aus einem Reservoir in dem Schlauchmaterial gezogen wird, wobei der Druckregler eingerichtet und ausgestaltet ist, um einen Druck der Sauerstoffquelle auf einen niedrigeren Druck zur Abgabe in das Schlauchmaterial (5) zu regeln, wobei das Schlauchmaterial (5) eine Öffnung einschließt, die eine Sauerstoffströmung durch diese hindurch drosselt, welche das Schlauchmaterial in vorgeschaltete und nachgeschaltete Abschnitte trennt, wobei der eine oder beide von dem Inhalationsgaseinlassdurchlass und Inhalationsgasauslassdurchlass eine Öffnung einschließt/einschließen, die eine Sauerstoffströmung durch diese hindurch drosselt, und wobei der nachgeschaltete Abschnitt des Schlauchmaterials so bemessen und die Öffnung(en) des Inhalationsgaseinlassdurchlasses und des Inhalationsgasauslassdurchlasses so dimensioniert ist/sind, dass eine vorbestimmte Minutenvolumenströmungsrate von Sauerstoff zu der tragbaren Gasverteilungsvorrichtung erreicht wird, wobei das Schlauchmaterial (5) eine Schlauchmaterialöffnung (1), um Sauerstoff von der Sauerstoffquelle über einen Schlauchmaterialeinlass (3) zu empfangen, ein von dem Schlauchmaterial (5) umschlossenes Volumen nachgeschaltet zu der Schlauchmaterialöffnung (1) und vorgeschaltet zu dem Konservierer einschließt, wodurch ein Schlauchmaterialreservoir für Sauerstoff definiert wird.

2. Sauerstoffabgabesystem nach Anspruch 1, wobei der Konservierer des Weiteren einen Haupteinlass (2) in Strömungskommunikation zwischen dem Schlauchmaterial (5) und dem Inhalationsgaseinlassdurchlass und Zeitwahlgaseinlassdurchlass umfasst, um so Sauerstoffströmungen über das Schlauchmaterial (5) und den Hauptkörpereinlass (2) zu empfangen.

3. Sauerstoffabgabesystem nach Anspruch 1, wobei der Konservierer des Weiteren einen Haupteinlass in Strömungskommunikation zwischen dem Schlauchmaterial (5) und dem Inhalationsgaseinlassdurchlass und Zeitwahlgaseinlassdurchlass umfasst, und der Zeitwahlgaseinlassdurchlass in nachgeschalteter Strömungskommunikation mit dem Schlauchmaterial (5) ist, um so eine Sauerstoffströmung über das Schlauchmaterial (5), jedoch nicht von dem Hauptkörpereinlass zu empfangen.

4. Sauerstoffabgabesystem nach Anspruch 1, wobei die tragbare Gasverteilungsvorrichtung eine Gesichtsmaske oder Nasenmaske ist, umfassend ein Inspirationsventil und ein Exspirationsventil, wobei der Konservierer in die Gesichtsmaske oder Nasenmaske integriert ist, der Inhalationsgaseinlassdurchlass und der Zeitwahlgaseinlassdurchlass in Fließkommunikation mit einem nachgeschalteten Ende des Schlauchmaterials (5) sind, und der Inhalationsgasauslassdurchlass sich in einen Innenbereich der Gesichtsmaske oder Nasenmaske öffnet.

5. Sauerstoffabgabesystem nach Anspruch 1, wobei die tragbare Gasverteilungsvorrichtung eine Nasenkanüle ist und der Konservierer inline mit dem Schlauchmaterial angeordnet ist.

6. Sauerstoffkonservierungssystem, wobei der Konservierer aus einem Kunststoffmaterial gefertigt ist.

7. Sauerstoffkonservierungssystem, des Weiteren umfassend eine Höhenanpassungsvorrichtung (A1, A2), die nachgeschaltet zu der Schlauchmaterialöffnung inline mit dem Schlauchmaterial angeordnet ist, die eingerichtet und ausgestaltet ist, um die vorbestimmte Minutenvolumenströmungsrate konstant zu halten, wenn eine Abnahme oder ein Anstieg des Umgebungsdrucks durch einen Anstieg oder eine Abnahme der Höhe verursacht wird.

8. Sauerstoffabgabesystem nach Anspruch 1, des Weiteren umfassend eine Höhenanpassungsvorrichtung (A1, A2), die nachgeschaltet zu der Schlauchmaterialöffnung inline mit dem Schlauchmaterial (5) angeordnet ist, die eingerichtet und ausgestaltet ist, um eine Minutenvolumenströmungsrate, die durch den Konservierer abgegeben wird, zu erhöhen oder herabzusetzen, wenn eine Abnahme beziehungsweise ein Anstieg des Umgebungsdrucks durch einen Anstieg beziehungsweise eine Abnahme der Höhe verursacht wird.

9. Sauerstoffabgabesystem nach Anspruch 8, wobei die Höhenanpassungsvorrichtung ein Gehäuse (UH, LH) mit einem Einlass, einem Auslass, mindestens einem Lüftungsloch an einem Ende davon, das in einen Außenbereich des Gehäuses und die Umgebungsatmosphäre öffnet, und einem Höhenanpassungsdiaphragma (D) umfasst, welches einen Innenbereich des Gehäuses in erste und zweite Regionen teilt, wobei die erste Region in Strömungskommunikation mit dem Gehäuseeinlass und Gehäuseauslass ist, die zweite Region in Strömungskommunikation mit jedem von dem einen oder mehreren Lüftungslöchern ist, das Höhenanpassungsdiaphragma mit einer Feder in eine Ruheposition vorgespannt ist, eine Abnahme des Umgebungsdrucks, die durch einen Anstieg der Höhe bewirkt wird, bewirkt, dass sich das Diaphragma gegen die Vorspannung der Feder bewegt, das Volumen der zweiten Region herabsetzt und das Volumen der ersten Region erhöht und demzufolge das Volumen des nachgeschalteten Schlauchmaterialabschnitts erhöht.

10. Sauerstoffabgabesystem nach Anspruch 8, wobei die Höhenanpassungsvorrichtung ein Segment des Schlauchmaterials (5) mit einer Flexibilität umfasst, die größer als diejenige der restlichen Segmente des Schlauchmaterials (5) ist, so dass eine Abnahme des Umgebungsdrucks, die durch einen Anstieg der Höhe bewirkt wird, bewirkt, dass das Segment des Schlauchmaterials, welches die Höhenanpassungsvorrichtung umfasst, auswärts expandiert und das Volumen des nachgeschalteten Schlauchmaterialabschnitts erhöht.

11. Sauerstoffabgabesystem nach Anspruch 1, wobei die Höhenanpassungsvorrichtung eingerichtet und ausgestaltet ist, um eine Sauerstoffmenge, die während eines Höhenanstiegs in die Schlauchmaterialeinlassöffnung strömt, zu erhöhen und eine Sauerstoffmenge, die während einer Höhenabnahme in die Schlauchmaterialeinlassöffnung strömt, herabzusetzen.

**Revendications**

1. Système d'administration d'oxygène comprenant une source d'oxygène, un régulateur de pression et un système de conservation d'oxygène pneumatique pour l'administration d'oxygène pour inhalation, comprenant un tube (5) qui est adapté et configuré pour recevoir du gaz d'une source de gaz, un dispositif de conservation (C) en communication d'écoulement en aval avec ledit tube (5), et un dispositif portable de distribution de gaz comprenant un masque facial (M), un masque nasal ou une canule nasale en communication d'écoulement en aval avec ledit dispositif de conservation qui est adapté et configuré pour administrer de l'oxygène à une personne pour inhalation, le dispositif de conservation comprenant un corps principal dans lequel est formé : un orifice de ventilation (45) qui débouche sur le corps principal ; une chambre asservie (19) divisée en régions supérieure (23) et inférieure (21) par un diaphragme asservi (25) ; une chambre de détection (31) divisée en régions supérieure (35) et inférieure (33) par un diaphragme de détection (37) ; un passage de détection d'inhalation qui s'ouvre dans le corps principal en communication d'écoulement en amont avec la région inférieure de la chambre de détection (33) ; un passage d'entrée de la chambre asservie (9) communiquant de manière fluide entre l'entrée du corps principal (3) et la région supérieure de chambre asservie (23) ; un passage de sortie de chambre asservie (47) en communication d'écoulement entre la région supérieure de chambre asservie (23) et le masque ou la canule nasale ; un passage d'entrée du gaz de synchronisation communiquant en amont avec la région inférieure de chambre asservie (21) ; et un passage de sortie du gaz de synchronisation communiquant entre la région inférieure de chambre asservie (21), la région supérieure de chambre de détection (35) et l'orifice de ventilation (45),

un chemin d'écoulement du gaz d'inhalation comprenant ledit passage d'entrée de la chambre asservie (9), ladite région supérieure de chambre asservie (23) et ledit passage de sortie de la chambre asservie (47) ;
un chemin d'écoulement du gaz de synchronisation étant constitué dudit passage d'entrée du gaz de synchronisation, de ladite région inférieure de chambre asservie (21), dudit passage de sortie du gaz de synchronisation et dudit orifice de ventilation (45) ;
le diaphragme asservi (25) étant sollicité jusqu'à une position fermée dans laquelle il obstrue la communication d'écoulement entre les passages d'entrée (9) et de sortie (47) de la chambre asservie et empêche l'écoulement de l'oxygène à travers le chemin d'écoulement du gaz d'inhalation ;

le diaphragme asservi (25) passant de sa position fermée à sa position ouverte lorsqu'une différence de pression, $P_{slave\ upper}$ - $P_{slave\ lower}$, entre les zones supérieure (23) et inférieure (21) de la chambre asservie, respectivement, dépasse une pression différentielle prédéterminée $P_{slave\ break}$ ;

le diaphragme de détection (37) étant sollicité dans une position fermée dans laquelle il obstrue la communication d'écoulement entre les passages d'entrée et de sortie du gaz de synchronisation (?) et empêche un flux d'oxygène à travers le chemin d'écoulement du gaz de synchronisation ; le diaphragme de détection (37) étant déplacé de sa position fermée à sa position ouverte lorsqu'une différence de pression, $P_{sensing\ upper}$ - $P_{sensing\ lower}$, entre les régions supérieure et inférieure de la chambre asservie secondaire, respectivement, dépasse une pression différentielle prédéterminée $P_{sensing\ break}$ ; et

le dispositif de conservation étant adapté et configuré de manière à ce que :

lorsque les diaphragmes de détection (37) et asservi (25) sont en position fermée, l'inhalation d'une personne portant le masque ou la canule nasale fait que $P_{sensing\ upper}$ - $P_{sensing\ lower}$ dépasse $P_{sensing\ break}$ et déplace le diaphragme de détection à sa position ouverte, l'oxygène s'écoule de l'orifice de ventilation (45) via l'entrée du corps principal (3) et les passages d'entrée et de sortie du gaz de synchronisation, et un flux d'oxygène sortant du passage de sortie du gaz d'inhalation est retardé d'une période de temps $\Delta T_1$ ;

à l'expiration de $\Delta T_1$, une diminution de la pression dans le chemin d'écoulement du gaz de synchronisation fait que $P_{slave\ upper}$ - $P_{slave\ lower}$ dépasse $P_{slave\ break}$ et le diaphragme asservi (25) est déplacé vers sa position ouverte, et l'oxygène s'écoule à travers le chemin d'écoulement du gaz d'inhalation tandis que les diaphragmes de détection (37) restent en position ouverte pendant une période de temps $\Delta T_2$ ;

à l'expiration de $\Delta T_2$, $P_{sensing\ upper}$ - $P_{sensing\ lower}$ tombe en dessous de $P_{sensing\ break}$ et le diaphragme de détection (37) est déplacé vers sa position fermée et l'oxygène est empêché de circuler dans le chemin d'écoulement du gaz de synchronisation tandis que l'oxygène continue de circuler dans le chemin d'écoulement du gaz d'inhalation pendant une période de temps $\Delta T_3$ ;

à l'expiration de $\Delta T_3$, une augmentation de la pression dans le chemin d'écoulement du gaz de synchronisation fait que $P_{slave\ upper}$ - $P_{slave\ lower}$ tombe en dessous de $P_{slave\ break}$ et le diaphragme asservi (25) est déplacé vers sa position fermée et les flux d'oxygène sont empêchés à travers les chemins d'écoulement du gaz de synchronisation et du gaz d'inhalation ; et

pendant $\Delta T_2$ et $\Delta T_3$, un bolus d'oxygène distribué par le dispositif de conservation au masque ou à la canule nasale est tiré d'un réservoir dans le tube, le régulateur de pression étant adapté et configuré pour réguler une pression de la source d'oxygène à une pression inférieure pour l'acheminer dans le tube (5), le tube (5) comprend un orifice bloquant le flux d'oxygène qui sépare le tube en parties amont et aval, l'un ou les deux passages d'entrée et de sortie du gaz d'inhalation comprennent un orifice bloquant le flux d'oxygène à travers eux, et la partie aval du tube est dimensionnée et le ou les orifices des passages d'entrée et de sortie du gaz d'inhalation sont dimensionnés de manière à atteindre un débit volumique minute prédéterminé d'oxygène vers le dispositif portable de distribution de gaz, le tube (5) comprenant un orifice de tube (1) pour recevoir de l'oxygène de la source d'oxygène via une entrée de tube (3), un volume enfermé par le tube (5), en aval de l'orifice de tube (1) et en amont du dispositif de conservation, définissant un réservoir de tube pour l'oxygène.

2. Système d'administration d'oxygène selon la revendication 1, le dispositif de conservation comprenant en outre une entrée principale (2) en communication d'écoulement entre le tube (5) et les passages d'entrée de gaz d'inhalation et de synchronisation de manière à recevoir des flux d'oxygène via le tube (5) et l'entrée du corps principal (2).

3. Système d'administration d'oxygène selon la revendication 1, le dispositif de conservation comprenant en outre une entrée principale en communication d'écoulement entre le tube (5) et les passages d'entrée de gaz d'inhalation et de synchronisation et le passage d'entrée de gaz de synchronisation étant en communication d'écoulement en aval avec le tube (5) de manière à recevoir un flux d'oxygène via le tube (5) mais pas à partir de l'entrée du corps principal.

4. Système d'administration d'oxygène selon la revendication 1, le dispositif portable de distribution de gaz étant un masque facial ou nasal comprenant une valve inspiratoire et une valve expiratoire, le dispositif de conservation étant intégré dans le masque facial ou nasal, les passages d'entrée de gaz d'inhalation et de synchronisation étant en communication fluide avec une extrémité en aval du tube (5), et le passage de sortie de gaz d'inhalation débouchant à l'intérieur du masque facial ou nasal.

5. Système d'administration d'oxygène selon la revendication 1, le dispositif portable de distribution de gaz étant une canule nasale et le dispositif de conservation étant disposé en ligne avec le tube.

**6.** Système de conservation de l'oxygène le dispositif de conservation étant constitué d'une matière plastique.

**7.** Système de conservation de l'oxygène comprenant en outre un dispositif d'ajustement de l'altitude (A1, A2) disposé en aval de l'orifice du tube, en ligne avec le tube, qui est adapté et configuré pour maintenir constant le débit volumique minute prédéterminé en cas de diminution ou d'augmentation de la pression ambiante causée par une augmentation ou une diminution de l'altitude, respectivement.

**8.** Système d'administration d'oxygène selon la revendication 1, comprenant en outre un dispositif de réglage de l'altitude (A1, A2) disposé en aval de l'orifice du tube en ligne avec le tube (5) qui est adapté et configuré pour augmenter ou diminuer un débit volumique minute distribué par le dispositif de conservation lorsqu'une diminution ou une augmentation de la pression ambiante, respectivement, est causée par une augmentation ou une diminution de l'altitude, respectivement.

**9.** Système d'administration d'oxygène selon la revendication 8, le dispositif d'ajustement de l'altitude comprenant un boîtier (UH, LH) ayant une entrée, une sortie, au moins un trou de ventilation sur une de ses extrémités qui s'ouvre vers l'extérieur du boîtier et l'atmosphère ambiante, et un diaphragme d'ajustement de l'altitude (D) divisant l'intérieur du boîtier en une première et une seconde région, la première région étant en communication d'écoulement avec l'entrée et la sortie du boîtier, la seconde région étant en communication d'écoulement avec chacun du ou des trous de ventilation, le diaphragme d'ajustement de l'altitude étant sollicitée par un ressort dans une position de repos, une diminution de la pression ambiante causée par une augmentation de l'altitude entraînant le déplacement du diaphragme contre la sollicitation du ressort, la diminution du volume de la seconde région et l'augmentation du volume de la première région et, par conséquent, l'augmentation du volume de la partie aval du tube.

**10.** Système d'administration d'oxygène selon la revendication 8, le dispositif d'ajustement de l'altitude comprenant une section du tube (5) ayant une flexibilité plus grande que les autres sections du tube (5) de sorte qu'une diminution de la pression ambiante causée par une augmentation de l'altitude entraîne la section du tube comprenant le dispositif d'ajustement de l'altitude à se dilater vers l'extérieur et à augmenter le volume de la partie en aval du tube.

**11.** Système d'administration d'oxygène selon la revendication 1, le dispositif de réglage de l'altitude étant adapté et configuré pour augmenter la quantité d'oxygène entrant dans l'orifice d'entrée du tube lors d'une augmentation de l'altitude et pour diminuer la quantité d'oxygène entrant dans l'orifice d'entrée du tube lors d'une diminution de l'altitude.

FIG 1

**FIG 2**

C

M

F

T

**FIG 3**

**FIG 4**

EP 3 582 836 B1

FIG 5

FIG 6

EP 3 582 836 B1

**FIG 7**

**FIG 8**

EP 3 582 836 B1

FIG 9

EP 3 582 836 B1

**FIG 10**

**FIG 11**

EP 3 582 836 B1

FIG 12

FIG 13

FIG 14

FIG 15

FIG 16

**FIG 17**

EP 3 582 836 B1

FIG 18

FIG 19

**FIG 20**

EP 3 582 836 B1

**FIG 21**

**FIG 22**

EP 3 582 836 B1

**FIG 23**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 648721 A
- US 25585816

- US 7089938 B